# EUROPEAN PATENT SPECIFICATION

(11) **EP 2 399 928 B1**
(45) Date of publication and mention of the grant of the patent: **08.11.2017**
(21) Application number: 10006541.6
(22) Date of filing: 23.06.2010
(51) Int. Cl.: C07K 14/005, C12Q 1/70

(54) **Specific TT virus sequences and chimeric TT virus host cell DNA molecules for use in diagnosis, prevention and treatment of cancer and autoimmunity**
Spezifische TT-Virus-Sequenzen und chimäre DNA-Moleküle von TT-Virus-Wirtszellen zur Verwendung bei Diagnose, Vorbeugung und Behandlung von Krebs und Autoimmunität
Séquences de virus TT spécifiques et molécules ADN à cellule hôte de virus TT chimérique à utiliser dans le diagnostic, la prévention et le traitement du cancer et auto-immunité

(43) Date of publication of application: 28.12.2011
(73) Proprietor: Deutsches Krebsforschungszentrum, 69120 Heidelberg (DE)
(72) Inventor: Zur Hausen, Harald, 69483 Waldmichelbach (DE); De Villiers, Ethel-Michele, 69483 Waldmichelbach (DE)
(74) Representative: Schüssler, Andrea

(56) References cited:
- WO-A2-00/28039
- WO-A2-00/46407
- WO-A2-01/42299
- WO-A2-03/023027
- WO-A2-2007/130519
- WO-A2-2008/138619
- JELCIC I ET AL: "Isolation of multiple TT virus genotypes from spleen biopsy tissue from a Hodgkin's disease patient: Genome reorganization and diversity in the hypervariable region", JOURNAL OF VIROLOGY, THE AMERICAN SOCIETY FOR MICROBIOLOGY, US, vol. 78, no. 14, 1 January 2004 (2004-01-01), pages 7498-7507, XP002463335, ISSN: 0022-538X, DOI: DOI:10.1128/JVI.78.14.7498-7507.2004 -& DATABASE EMBL [Online] 3 February 2009 (2009-02-03), "Torque teno virus, isolate tth25, complete genome", XP002610817, retrieved from EBI accession no. AJ620222 Database accession no. AJ620222
- PENG Y H ET AL: "Analysis of the entire genomes of thirteen TT virus variants classifiable into the fourth and fifth genetic groups, isolated from viremic infants", ARCHIVES OF VIROLOGY, vol. 147, no. 1, January 2002 (2002-01), pages 21-41, XP002610938, ISSN: 0304-8608

## Description

### FIELD OF THE INVENTION

The present invention relates to single-stranded new sequences of a specific TT virus sequence and host cell DNA that are capable of replicating autonomously for use as gene vectors and artificial chromosomes.

### BACKGROUND OF THE TECHNOLOGY

Since their discovery in 1997 by Okamoto and colleagues TT viruses (TTV) have been found to be widely spread in all human populations, in domestic animals, and in old world primates (1,2). A large number of types and pseudotypes have been identified in humans, pointing to a remarkable heterogeneity of this virus family now being assigned as a new virus family, *Anelloviridae* (3). Viral DNA can be demonstrated in sera of almost every human being and some reports even document such DNA in newborn children and cord blood, suggesting prenatal transmission of these agents (4,5). In spite of the widespread occurrence of these viruses, intensive research performed during more than 10 years failed to demonstrate a pathogenic role of such infections in human disease.

TT viruses have not been successfully replicated in human tissue culture cells, although indications exist that replication can be achieved in human cells of epithelial or hematopoietic origin. In the latter, replicative cycles of herpes group viruses (Epstein-Barr virus) seem to exert an enhancing effect for the amplification of latent or transfected TTV genomes (6). In addition, TT viruses frequently reveal intramolecular rearrangements which lead to subviral DNA genomes in part defective and with novel open reading frames. They replicate autonomously over prolonged periods of time in infected tissues (7). These subviral DNAs are found in normal and malignant human biopsy materials. A TTV highly conserved region and nested primers to detect a 71 nucleotide fragment of said highly conserved region is disclosed in Peng, Y.H. et al.; Archives of Virology, 147(1):21-41, 2002.

During the past years, some data have been compiled indicative of an association of TT virus infection with human malignant tumors. A high rate of TT virus load has been noted in a spleen biopsy of a patient with Hodgkin's lymphoma (24 individual TTV genotypes) (8). Similarly, other reports describe a higher rate of TTV prevalence in colorectal and esophageal cancer and in hematopoietic malignancies in comparison to non-tumorous tissue from the same or other patients (9,10). Yet, the ubiquity of these infections rendered an interpretation of these results rather difficult and did not permit a linkage of these observations with tumor development.

WO 00/46407 discloses primers or probes usesful for the detection of TTV and vectors based on full-length TTV. WO 2007/130519 describes hybridization microarrays for detecting viruses. WO 03/023027 discloses DNA sequences comprising the genome of tumor-associated TT virus and WO 2008/138619 discloses a rearranged TTV polynucleic acid associated with childhood leukaemia.

### SUMMARY OF THE INVENTION

The technical problem underlying the present invention is to identify specific TTV sequences that might be clearly associated with diseases like cancer or autoimmune diseases and, thus, to provide means for diagnosis and therapy.

The solution to said technical problem is achieved by providing the embodiments characterized in the claims. Recent observations of the persistence of a 71 base highly conserved region (HCR), present with only minor variation in all TTV isolates characterized thus far, in a larger number of human cancer and immortalized cell lines were somewhat surprising in view of the regular long-time non-permissibility of the same cells for transfected TTV DNA. Even more surprising has been the linkage of host cell DNA sequences in an apparently single-stranded form to the TTV-HCR. The frequency and regularity of persistence of this obviously extrachromosomal genetic material in a large number of cancer cell lines as well as in biopsies of affected brain tissue from patients with multiple sclerosis prompted the following hypothesis: Host cell genes either modified in the recombinatory process or dys-regulated by novel TTV regulatory sequences play a significant role in human carcinogenesis and also in some autoimmune reactions. They may even replace or, in a certain sense, functionally correspond to retrovirus infections in rodent and chicken cells.

### A novel role for TT viruses in human cancer and autoimmunity

The surprising observation of host cell DNA linked to an apparently single-stranded form to TT virus HCR is the basis for the following conclusion: TT viral sequences have not yet been demonstrated as integrated into double-stranded cellular DNA, persisting within host cell chromosomes. Thus, the opposite finding of host cell DNA, linked in a single-stranded state to the TTV HCR should have biological significance. The present data indicate their long-time persistence as episomes in human cancer cell lines, pointing to a role of this persistence in cell proliferation. Two aspects seem to require specific consideration: a possible role of those recombinants in cancer and in autoimmunity.

One possibility is the random integration of host cell sequences into TTV episomes. This may happen after strand displacement in the course of aberrant DNA replication or after reverse transcription of cellular RNA. In case of random integration a larger number of recombinants should be innocuous and harmless for cells carrying these recombinants. A growth-promoting property of transcripts of the TTV HCR, as well as integration and transcription of growth-stimulating host cell genes, their modification in the process of integration or their dysregulation by the TTV HCR however, will result in proliferative consequences. These episomes should acquire immortalizing and under certain conditions transforming properties. In combination with additional modifications of the host cell genome they may direct malignant growth. This mode of action reveals a distant resemblance to the insertion of cellular oncogenes into retroviral genomes.

### The TTV-Oncogene concept

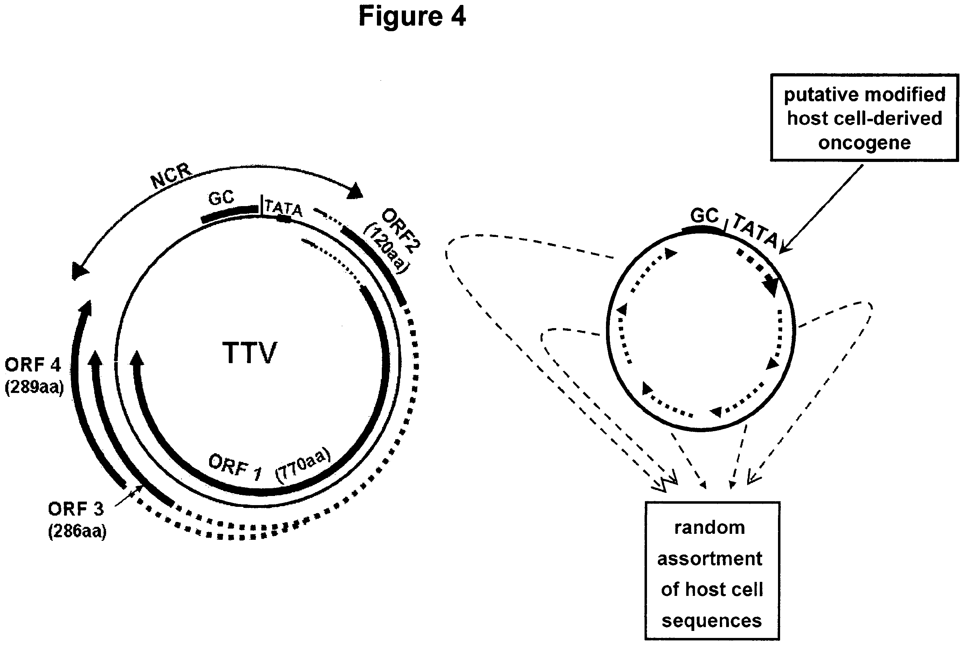

The previous considerations are summarized in Fig 4. Obviously, the recombination between the TTV regulatory region and cellular nucleic acids must be a relatively frequent process, since such recombinants are found in the majority of cell lines thus far analyzed. It also should contribute to cell proliferation, otherwise the regular persistence of such molecules, in part over decades of continuous proliferation, would be difficult to explain. It is assumed that this type of recombination is a random process, involving different types of cellular genes. The coding function of the TTV HCR and/or the uptake of genes steering cell proliferation, or blocking the function of proliferation antagonists, or inhibiting cell differentiation should lead to an accumulation of cells containing these types of recombinants. It is envisaged that this, in combination with additional mutational or recombinational events of the cells harbouring such TTV-host cell nucleic acid recombinants, provides a selective advantage for cells carrying such episomes. The presence of the latter would represent a prime risk factor for malignant conversion. In this sense those recombinations should be of general importance for different types of human cancers, although a certain degree of specificity for a limited set of genes would be expected for individual cancer types.

The implications of this model are profound. They reach from cancer prevention, early detection into cancer therapy. The important role of TTV infections and of the persistence of TTV HCR is stressed by the available information. Prevention of these infections should reduce the risk for the development of the described recombinants. The diagnosis of specific recombinants would probably contribute to cancer risk assessment. Profound implications would be expected for cancer therapy: the TTV HCR emerges as the prime determinant for the persistence and maintenance of the single-stranded episomes. Since this region appears to be part of an open reading frame, it should be vulnerable to small interfering RNAs or DNAs. Thus, it offers a suitable target for future therapeutic deliberations.

Two other aspects deserve discussion: certain parallels which seem to exist to retroviral carcinogenesis in rodents and chicken and the use of autonomously replicating TTV-based vector systems for gene therapy. Insertional mutagenesis, the uptake and modification of cellular growth-stimulating genes, rendering them into oncogenes has frequently been analyzed in animal systems. This has thus far not been reported for human cancers. Do TT viruses replace this niche in human and other primate cells? Do TTV compete successfully with retrovirus infections in taking over their role in specific species? The episomal persistence of single-stranded DNA, however, emerges as a remarkable difference to retrovirus-induced carcinogenesis.

Autonomously replicating subviral DNA molecules of approximately 400 bases of TTV origin have been described before (11). It is tempting to speculate that they or specific TTV-host cell recombinants may represent optimal vector systems for future approaches in gene therapy and for the construction of artificial chromosomes.

### The recombinant TTV-host cell DNA autoimmunity concept

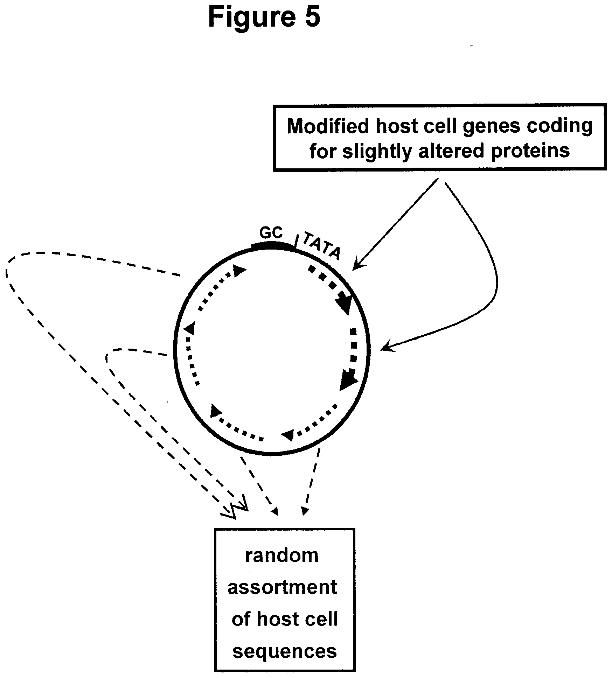

The existence of TTV host cell nucleic acid recombinants also permits a novel view on aspects of autoimmune diseases and other chronic diseases (potentially even conditions like arteriosclerosis and Alzheimer's disease). Modification or dys-regulation of cellular proteins may originate from insertional events of cellular genes into single-stranded DNA or to the different HCRs exerted by TTV elements (Fig. 5). They could provide a convenient explanation for autoimmune reactions, even for local ones, like in multiple sclerosis (MS) or Crohn's disease. In the latter two cases in particular, the reactivation of other local infections (potentially herpes-type viruses) would provide a stimulus for the local amplification and gene activity of the respective TTV-host cell nucleic acid recombinants. In MS, this could explain recurrent episodes of disease progression. A model of the autoimmunity concept is depicted in Figure 5.

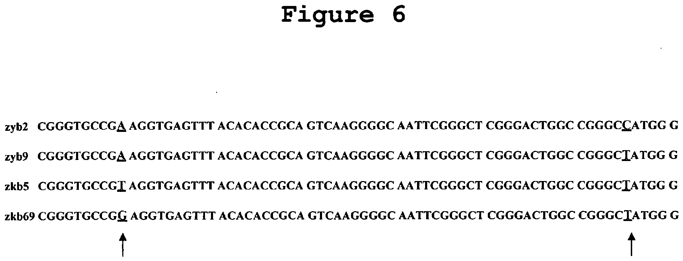

Similarly, rearranged TT virus molecules of 719, 642, and 621 bases have been identified which replicate autonomously upon transfection of specific cell lines. Their DNA composition and derivation from specific complete TTV genotypes is shown in Figure 6. Here the rearrangement results in novel open reading frames in part with epitopes related to those of juvenile diabetes and rheumatoid arthritis.

### Conclusion

The models of the present invention for a role of TTV-host cell nucleic acid recombinants is based on the demonstration of the single-stranded chimeric molecules between the TTV HCR and host cell DNA and rearranged autonomously replicating TTV molecules of substantially reduced molecular weights. Both, the TTV oncogene concept and the TTV autoimmunity concept will clearly provide novel approaches to prevention, diagnosis, and in particular to therapy of these conditions and will improve the prognosis of the respective patients.

Thus, the present invention relates to a TT virus polynucleic acid, wherein the TT virus polynucleic acid consists of
(a) a 71 base nucleotide sequence (HCR) shown in Figure 6;
or(b) a nucleotide sequence which is the complement of the nucleotide sequence of (a).

The present invention provides an expression vector comprising a TT virus polynucleic acid (HCR) of the invention operatively linked to prokaryotic or eukaryotic transcription and translation control elements as well as a host cell comprising the expression vector.

### DETAILED DESCRIPTION OF THE INVENTION

### Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The term *"biological sample"* as used herein refers to a sample that may be extracted, untreated, treated, diluted or concentrated from an animal. Biological sample refers to any biological sample (tissue or fluid) containing a TTV polynucleic acid of the invention and refers more particularly to blood serum samples, plasma samples, biopsy samples, cerebrospinal fluid samples etc..

By *"carrier" is* meant any substance of typically high molecular weight to which a non- or poorly immunogenic substance (e.g., a hapten) is naturally or artificially linked to enhance its immunogenicity.

The term *"diagnosis"* is used herein in its broadest sense to include detection of an antigen reactive to a sub-immunoglobulin antigen-binding molecule. Also included within its scope, is the analysis of disorder mechanisms. Accordingly, the term "diagnosis" includes the use of monoclonal antibodies for research purposes as tools to detect and understand mechanisms associated with a disease or condition of interest. It also includes the diagnostic use of TTV polynucleic acid of the invention for the detection of homologous or complementary RNA transcribed from such molecules.

The term "*immunogenicity*" is used herein in its broadest sense to include the property of evoking an immune response within an organism. Inmunogenicity typically depends partly upon the size of the substance in question, and partly upon how unlike host molecules it is. It is generally considered that highly conserved proteins tend to have rather low immunogenicity.

The term "*patient*" refers to patients of human or other mammal origin and includes any individual it is desired to examine or treat using the methods of the invention. However, it will be understood that '*patient*" does not imply that symptoms are present. Suitable mammals that fall within the scope of the invention include, but are not restricted to, primates, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes).

By *'pharmaceutically acceptable carrier*" is meant a solid or liquid filler, diluent or encapsulating substance that may be safely used in any kind of administration.

The term "*related disease or condition"* is used herein to refer to a disease or condition that is related anatomically, physiologically, pathologically and/or symptomatically to a reference disease or condition. For example, diseases or conditions may be related to one another by affecting similar anatomical locations (e.g., affecting the same organ or body part), affecting different organs or body parts with similar physiological function (e.g., the oesophagus, duodenum and colon which rely an peristalsis to move food from one end of the alimentary canal to the other), by having similar or overlapping pathologies (e.g., tissue damage or rupture, apoptosis, necrosis) or by having similar or overlapping symptoms (i.e., allergic response, inflammation, lymphocytosis). Thus, for example, an antigen associated with ulcerated colitis may also be associated with perforation of the colon because these disease affects the same organ (i.e., colon).

The term "*treating*" is used herein in its broadest sense to include both therapeutic and prophylactic (i.e., preventative) treatment designed to ameliorate the disease or condition.

The term *"episome"* is used herein to refer to a portion of genetic material that can exist independent of the main body of genetic material (chromosome) at some times or continuously and replicate autonomously, while at other times is able to integrate into the chromosome. Examples of episomes include insertion sequences, transposons and the TTV of the invention.

### FIGURE LEGENDS

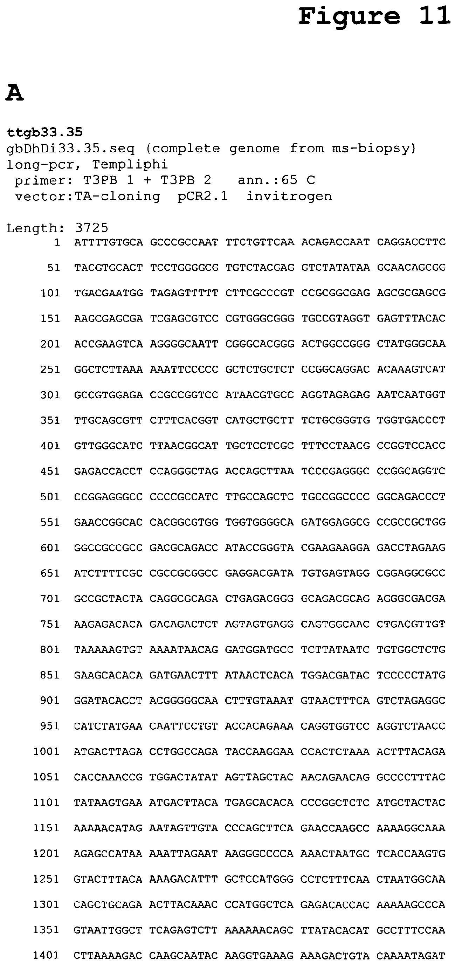

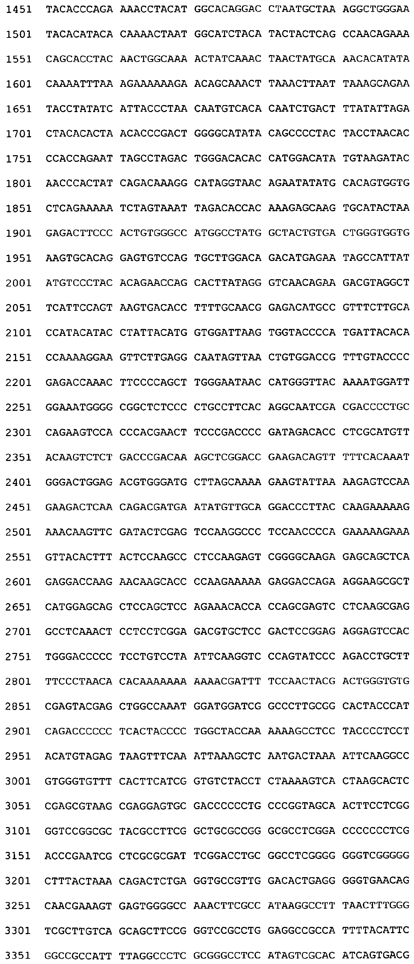

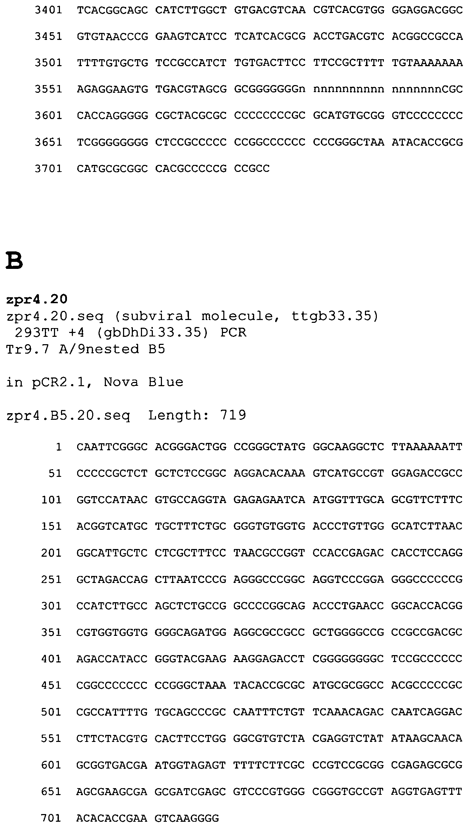

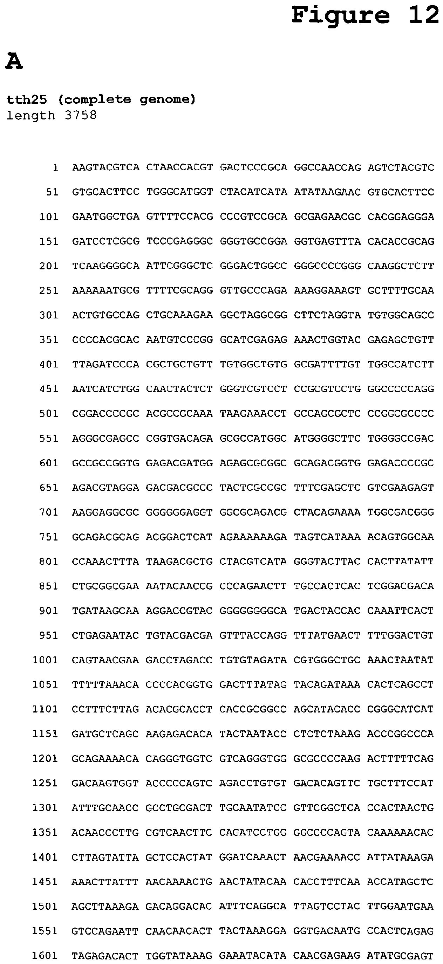

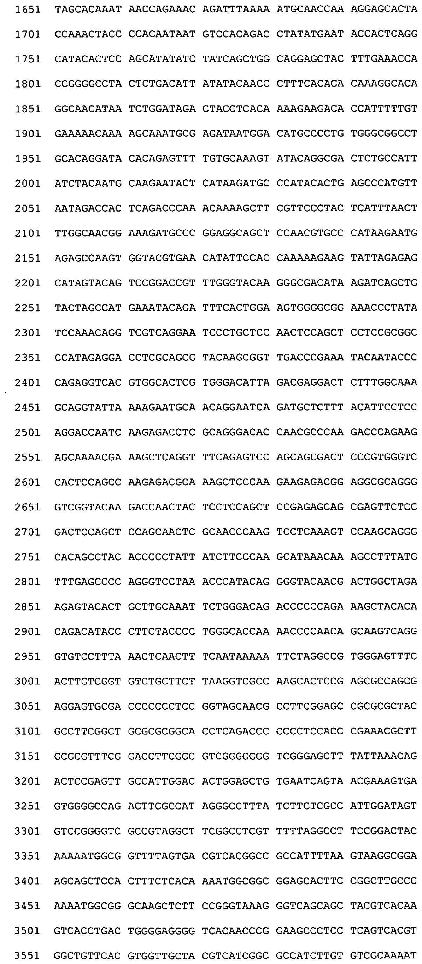

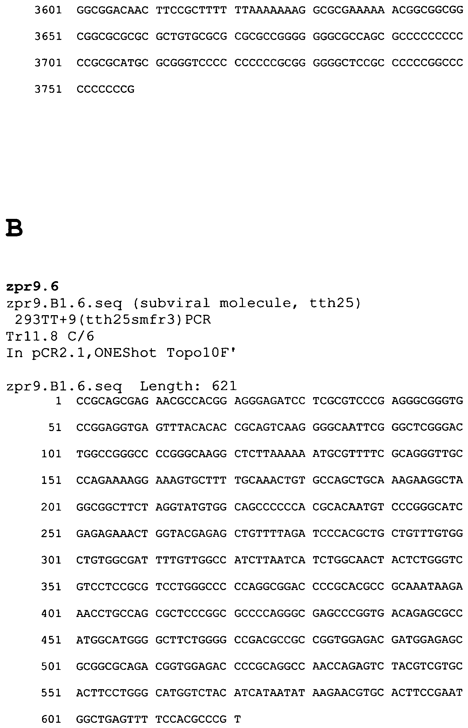

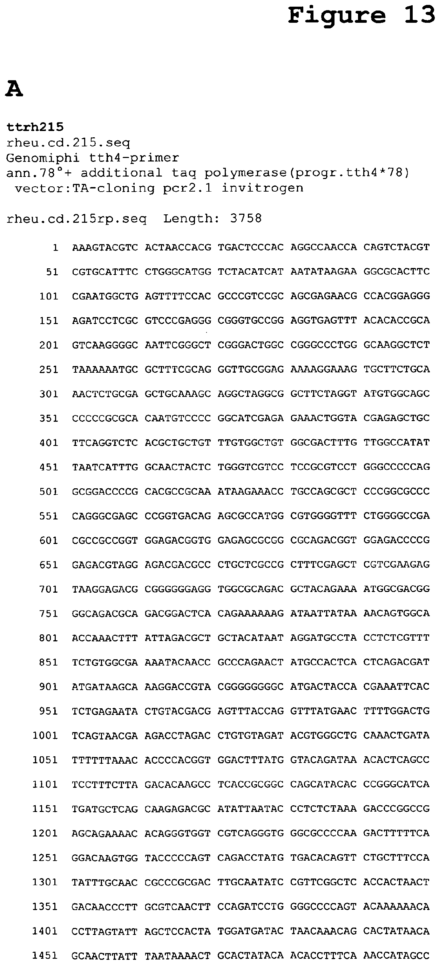

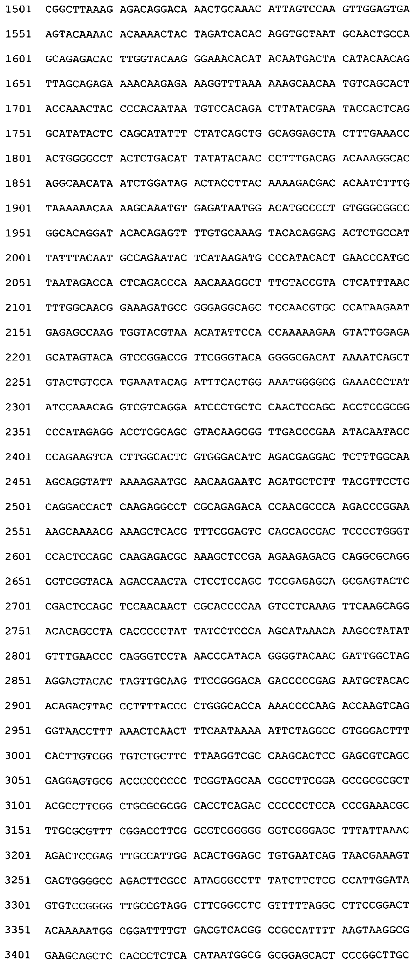

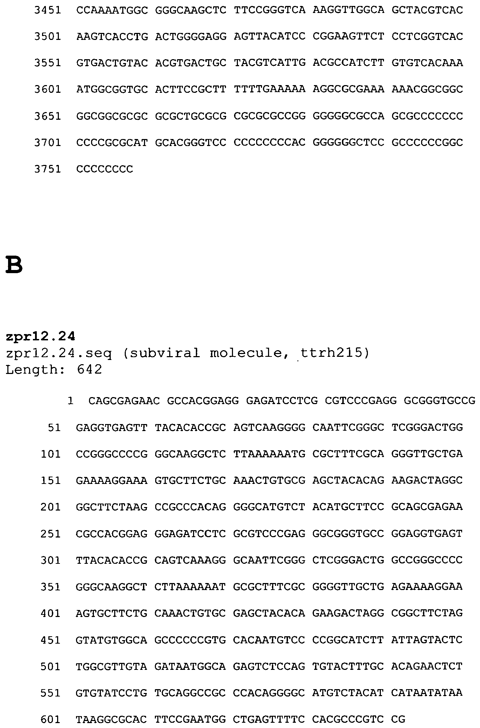

Figure 1: PCR amplification of a 71 base fragment containing the highly conserved TTV region (HCR) in 4 different cell lines, *L1236* (EBV-negative Hodgkin's lymphoma line), *HSB-2* (acute lymphoblastic leukemia line), *KR* and *IGL* (melanoma cell lines) and placenta DNA
Figure 2: Spooled DNA remaining in the supernatant of L1236 cells after precipitation and removal of high molecular weight DNA and RNase digestion
   Two bands are visible in the region between 4.3 and 6.6 base bands.
Figure 3: Outwards-directed long-PCR, using primers of the 71 base TTV HCR region in HSB-2 DNA
   Two bands are visible in regions corresponding to 4.5 to 7 kb. In addition, bands emerge in the region corresponding to 0.4 to 0.7 kb.
Figure 4: Schematic outline of the TTV oncogene concept
   The left part represents the genomic organization of wild-type TTV genomes. The right part envisages the integration of host cell DNA into the single-stranded plasmids.
Figure 5: Schematic outline of the TTV host cell DNA autoimmunity concept
   The modified host cell genes should code for immuno-reactive antigenic epitopes.
Figure 6: PCR amplification of the 71 base HCR from the DNA of 4 different cell lines
   The arrows point to the two sites with variations in the nucleotide sequences.
Figure 7:
   **(A)** The autonomously replicating 719 base TTV DNA (right) and the complete TTV sequence from which it is derived. The nucleotide composition of both molecules is found in Figure 11A+B.
   **(B)** The autonomously replicating 621 base TTV DNA (right) and the complete DNA sequence from which it is derived. The nucleotide composition of both molecules is found in Figure 12A+B.
   **(C)** The autonomously replicating 642 base TTV DNA (right) and the complete DNA sequence from which it is derived. The nucleotide composition of both molecules is found in Figure 13A+B.

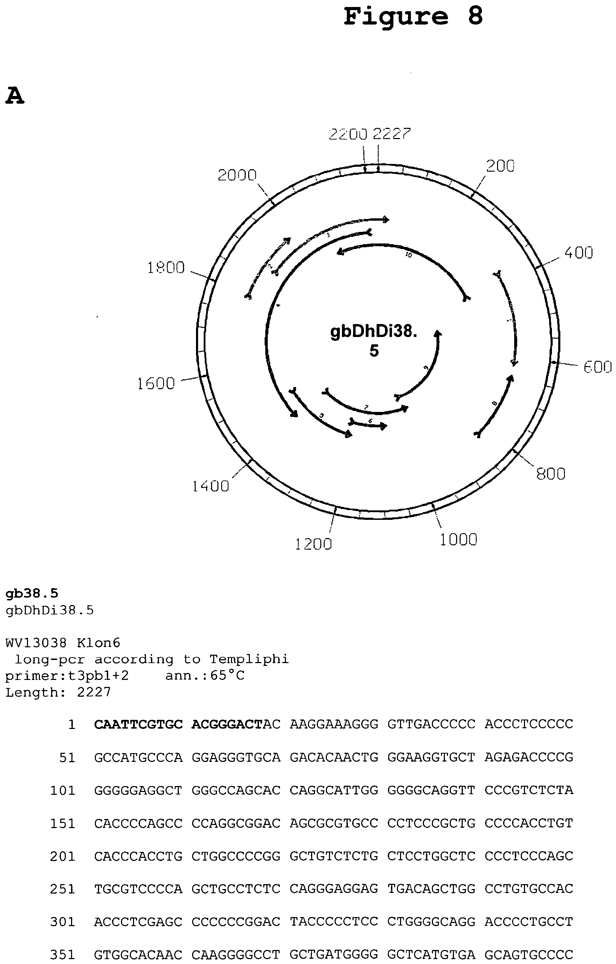

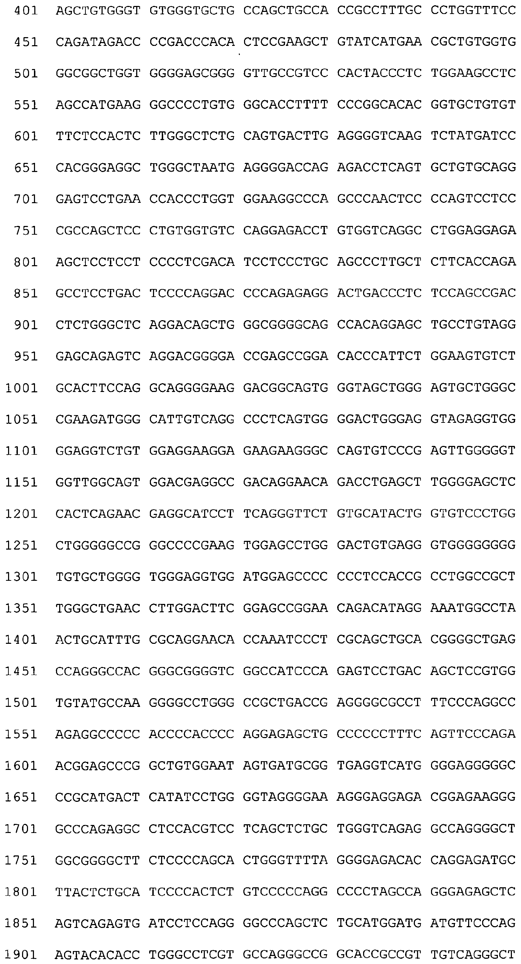

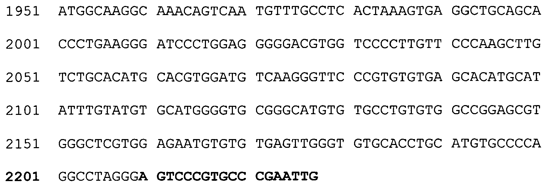

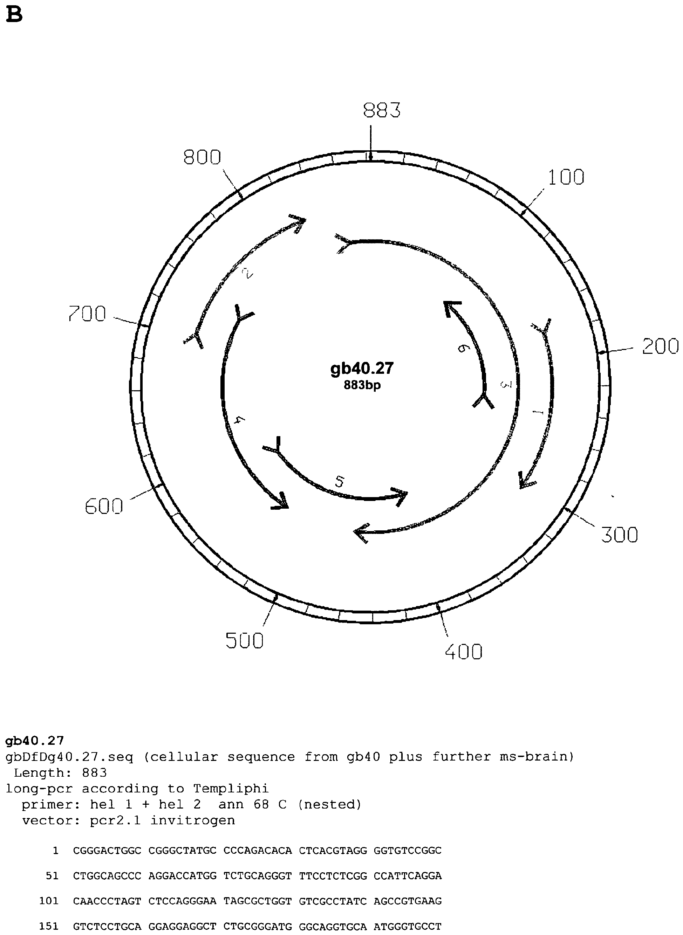

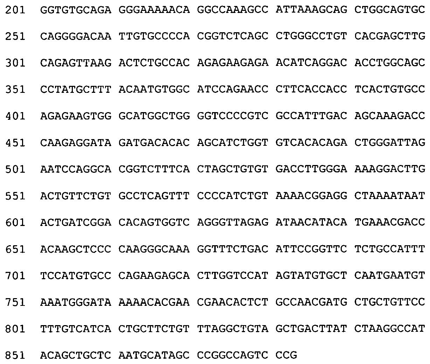

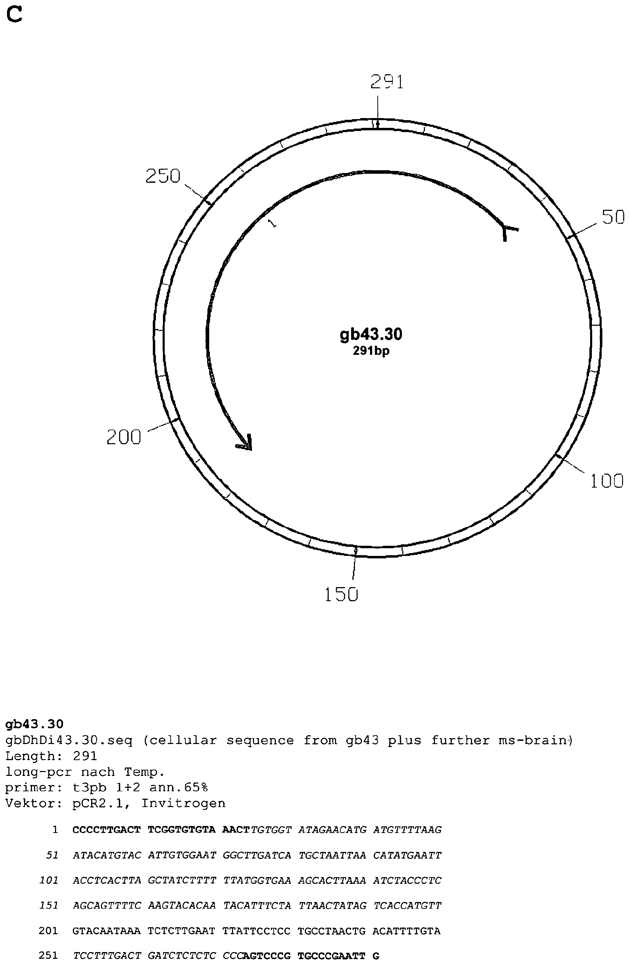

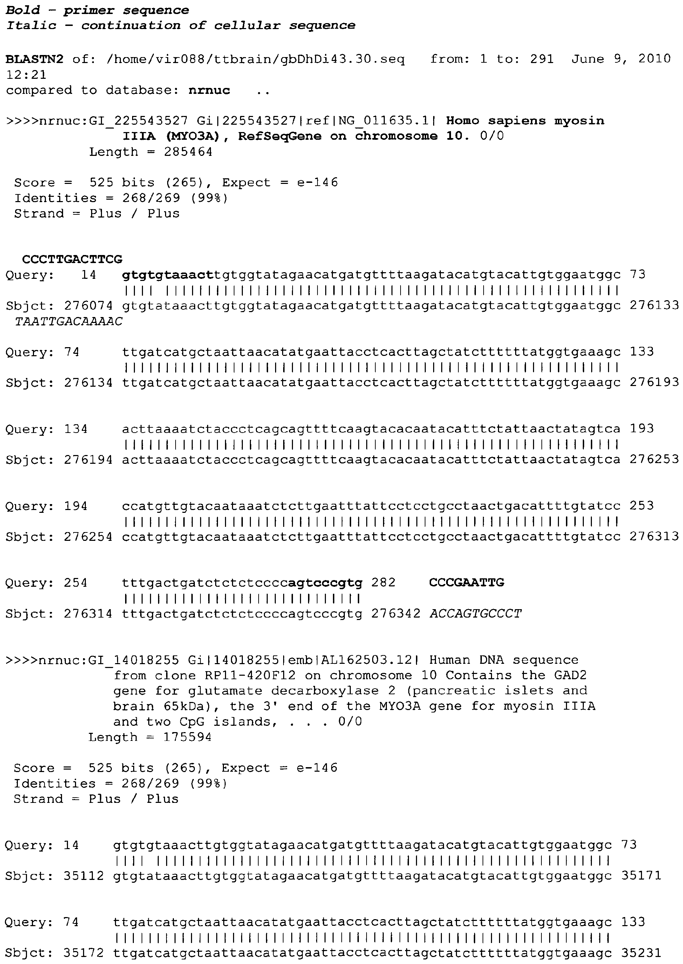

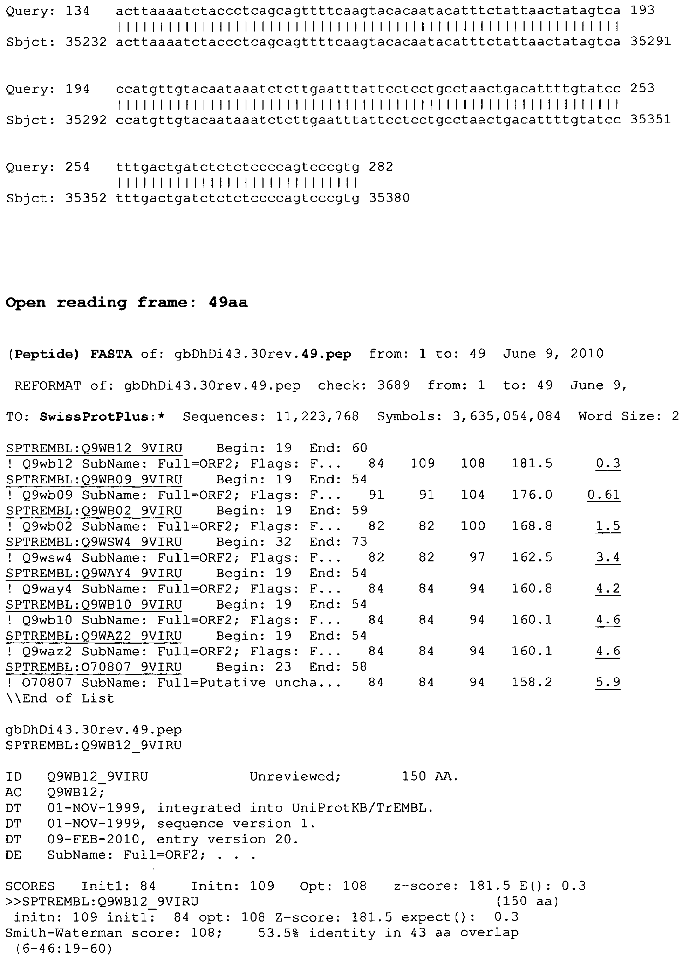

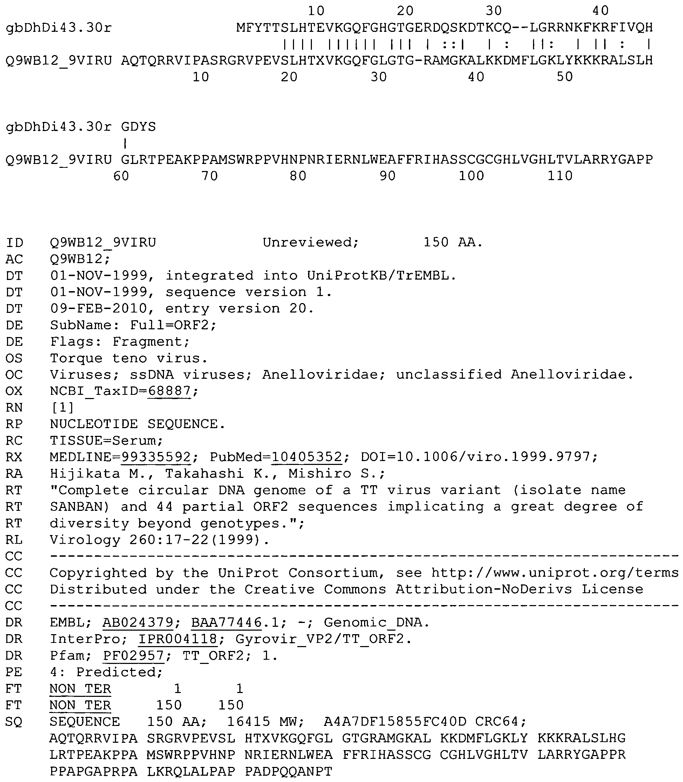

Figure 8: Three exemplary chimeric TTV / truncated host cell DNA sequences from brain biopsies of patients with multiple sclerosis
   **(A)** Chimeric cellular sequences derived from chromosome 1 with some homologies to prion and Wilms tumor sequences and the 3' end of myeloid lymphoid leukemia 3 (MLL3) pseudogene. Human DNA sequence from clone RP11-14N7 on chromosome 1. Contains 3'end of a myeloid/lymphoid or mixed lineage leukemia 3 (MLL3) pseudogene, a seven transmembrane helix receptor pseudogene, the 5'-end of a novel gene.
   **(B)** Chimeric cellular sequences derived from chromosome 16. Homologies to transcription factor 3 (TF 3C), protein signatures for chemokine receptors and leukotriene B4 receptor.
   **(C)** Chimeric cellular sequences derived from chromosome 10, truncated sequence of myosin, reactivity reported for multiple sclerosis patients and those with rheumatoid arthritis (sequence contains both full primers front and back).

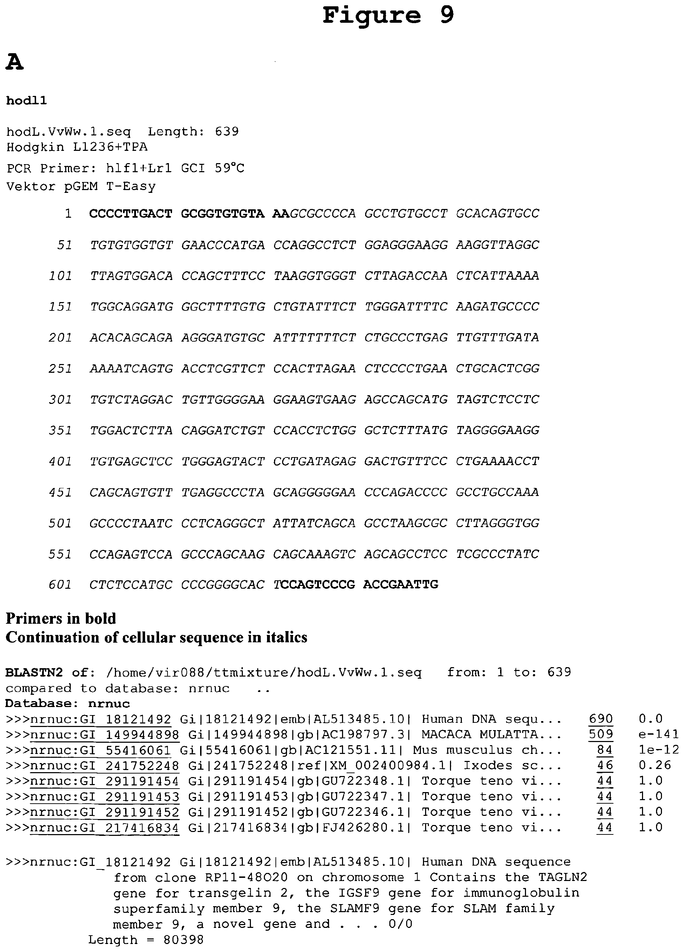

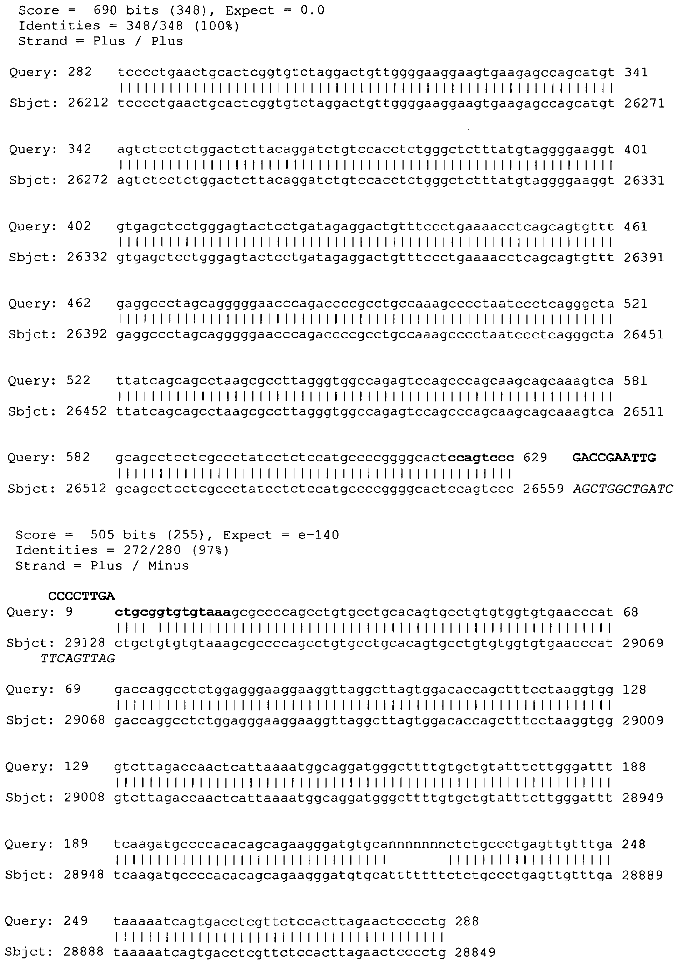

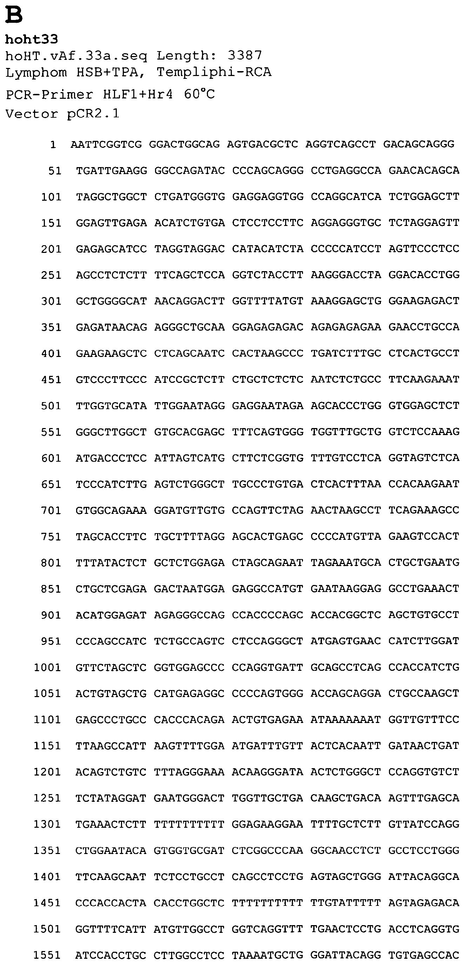

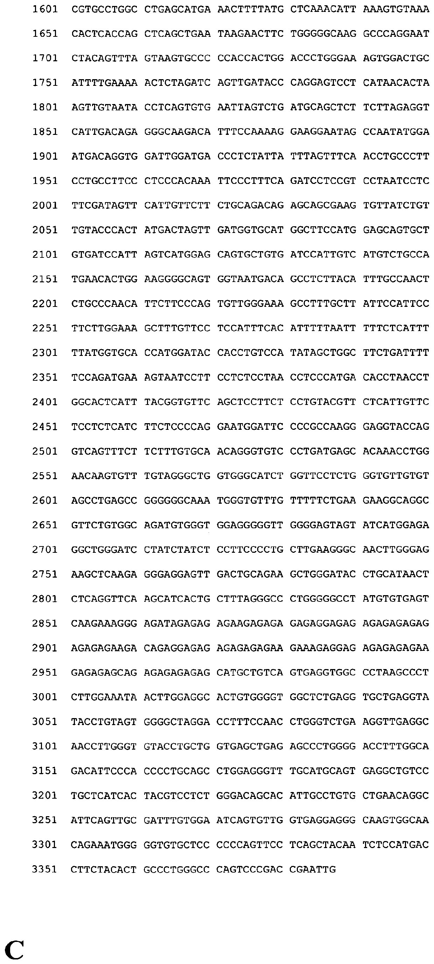

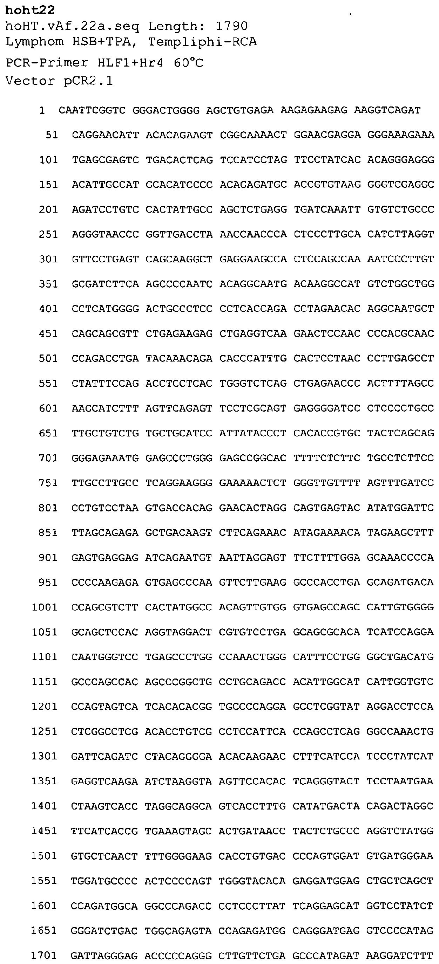

Figure 9: Three exemplary chimeric TTV/truncated host cell DNA sequences from cell lines derived from patients with Hodgkin's disease or leukemia
   **(A)** Chromosome 1 sequences with part of transgelin 2, the IGSF9 gene for immunoglobulin superfamily member 9, the SLAM9 gene.
   (B) Translated protein sequences with substantial homology to the oncogenes v-myb (avian myeloblastosis viral oncogene), but also to c-myb. This sequence was amplified with the forward primer at both ends.
   **(C)** Derived from chromosome 10. High homology with "Deleted in malignant 1 Protein" (DMBT), an identified tumor suppressor gene. This sequence was amplified with the forward primer at both ends.

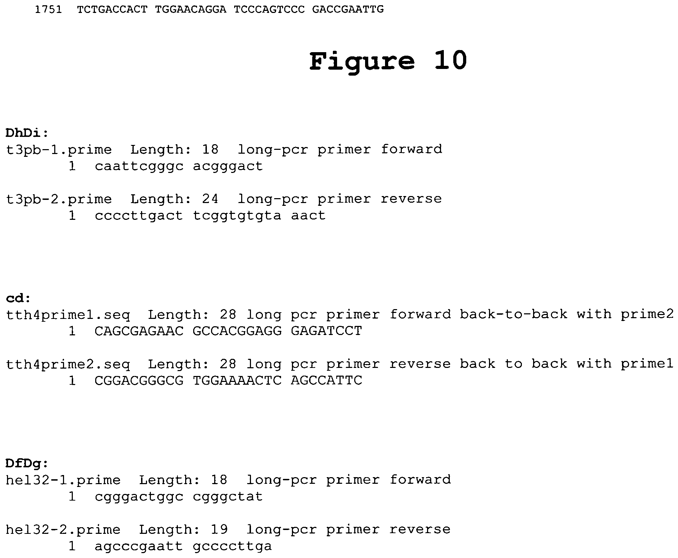

Figure 10: Primer sequences used in the reactions described in the Examples, derived from the 71 base HCR.
Figure 11:
   **(A)** Complete TTV sequence from which autonomously replicating 719 base DNA has been obtained.
   **(B)** Complete sequence of the autonomously replicating 719 base TTV DNA.
Figure 12:
   **(A)** Complete TTV sequence (tth25) from which autonomously replicating 621 base DNA has been obtained.
   **(B)** Complete sequence of the autonomously replicating 621 base TTV DNA.
Figure 13:
   **(A)** Complete TTV sequence (ttrh215) from which autonomously replicating 642 base DNA has been obtained.
   **(B)** Complete sequence of the autonomously replicating 642 base TTV DNA.

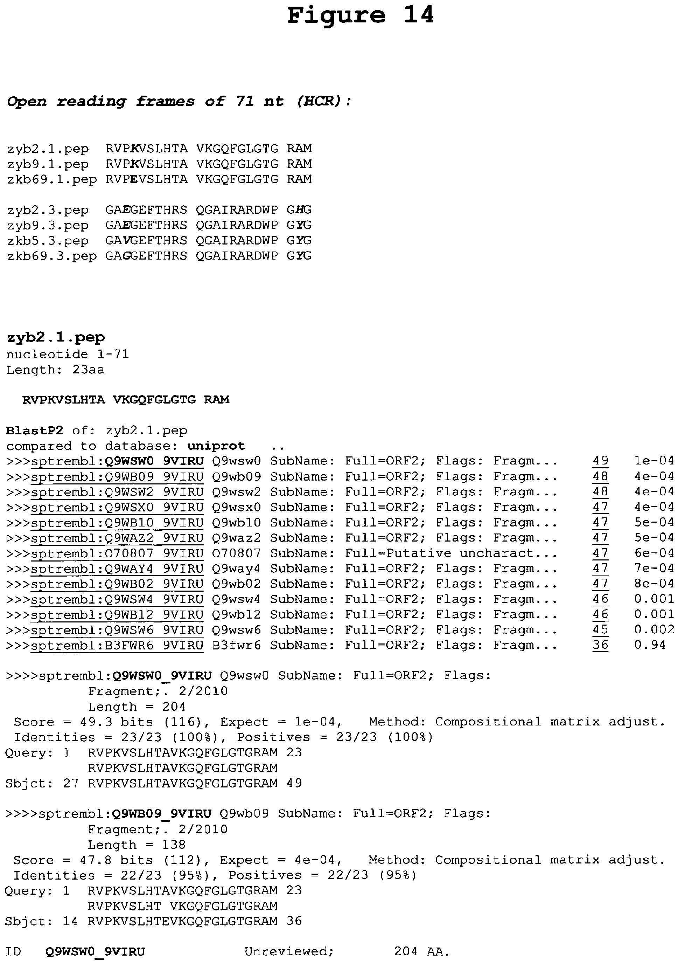

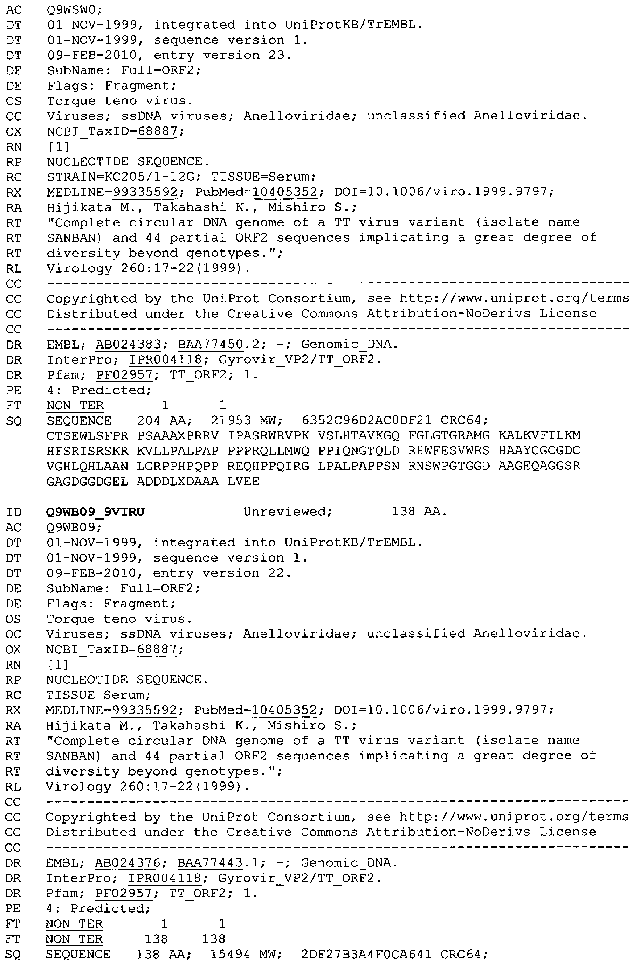

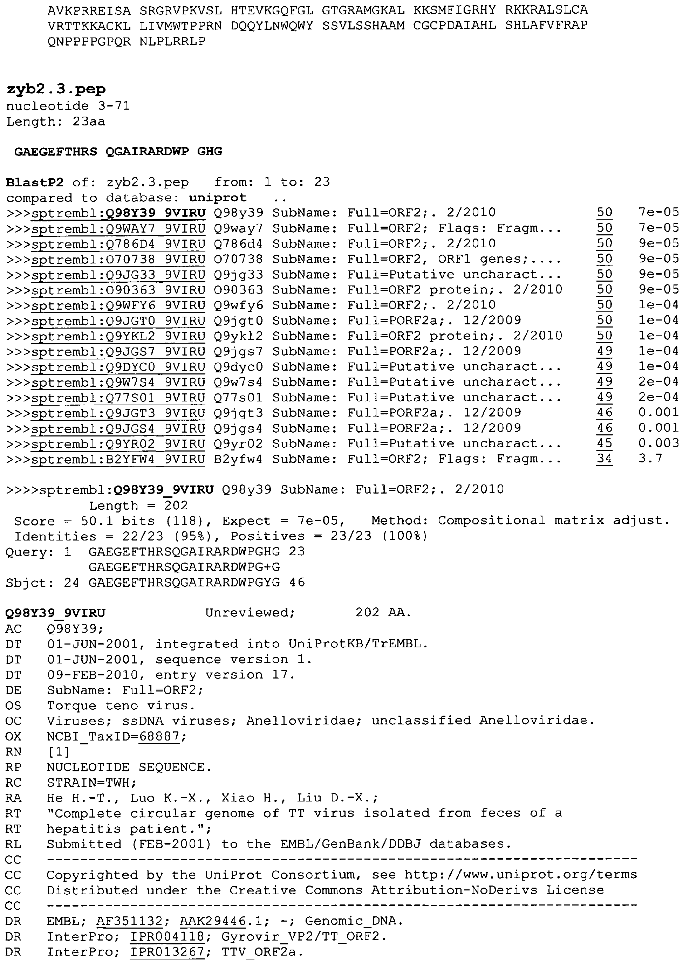

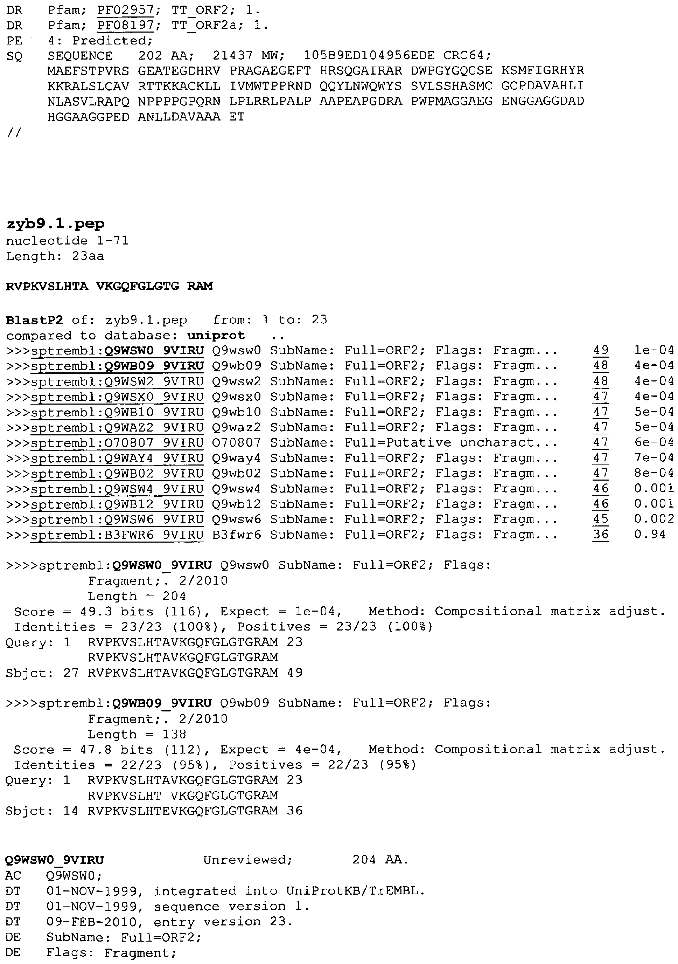

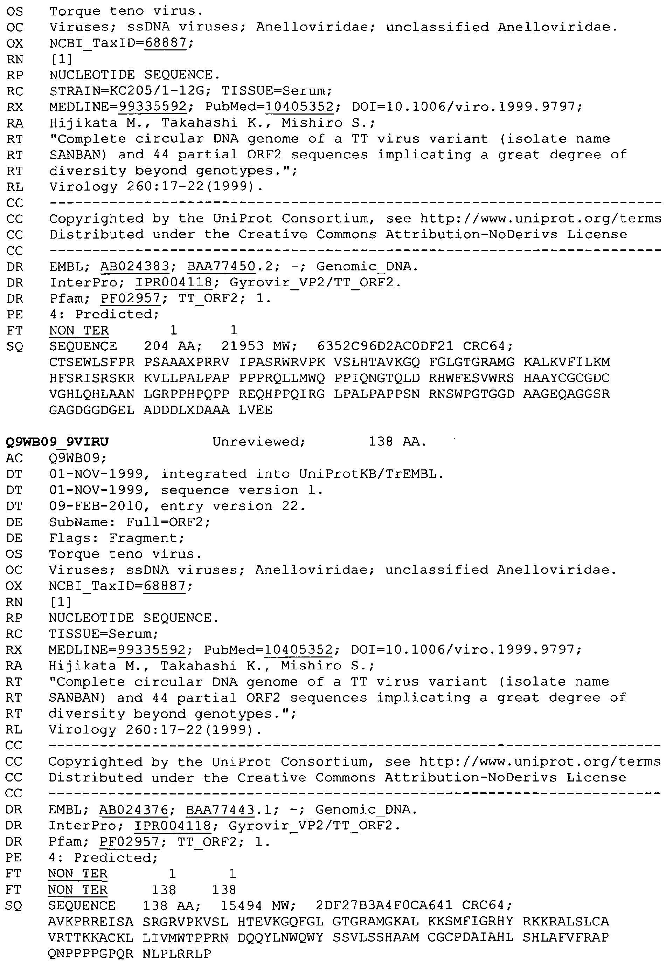

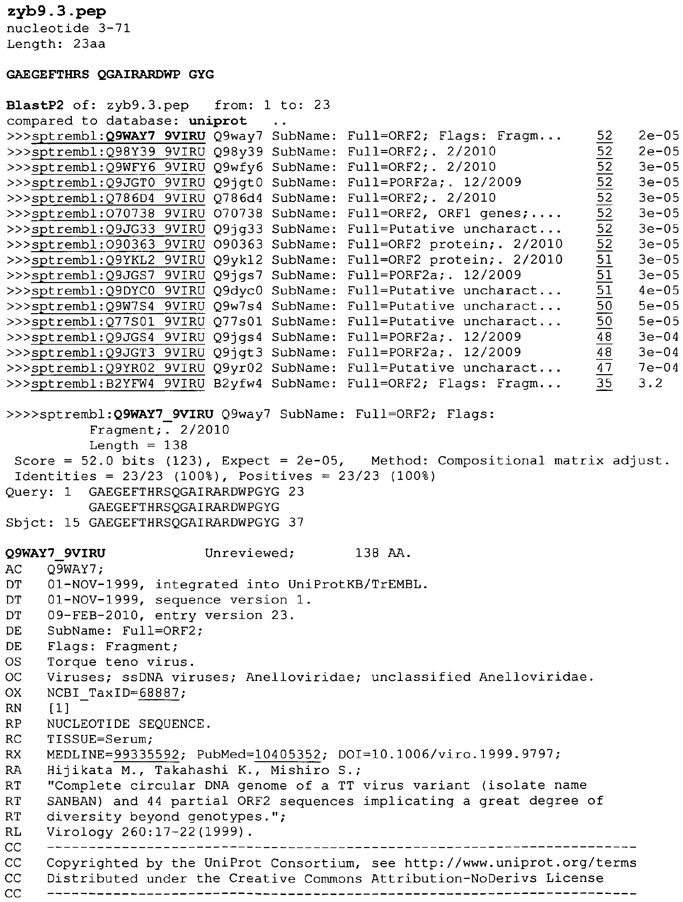

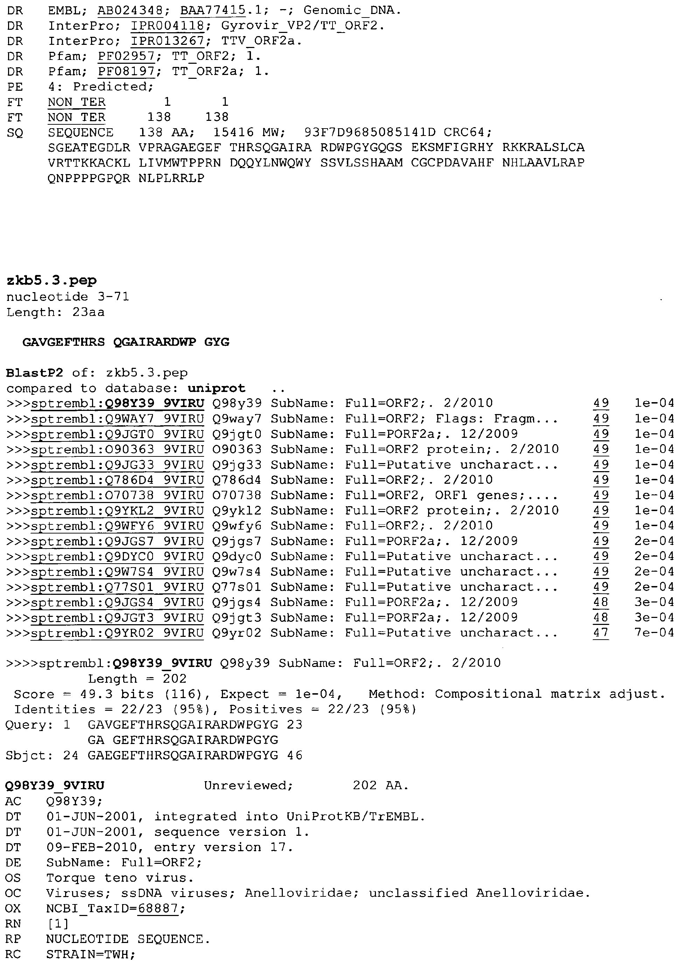

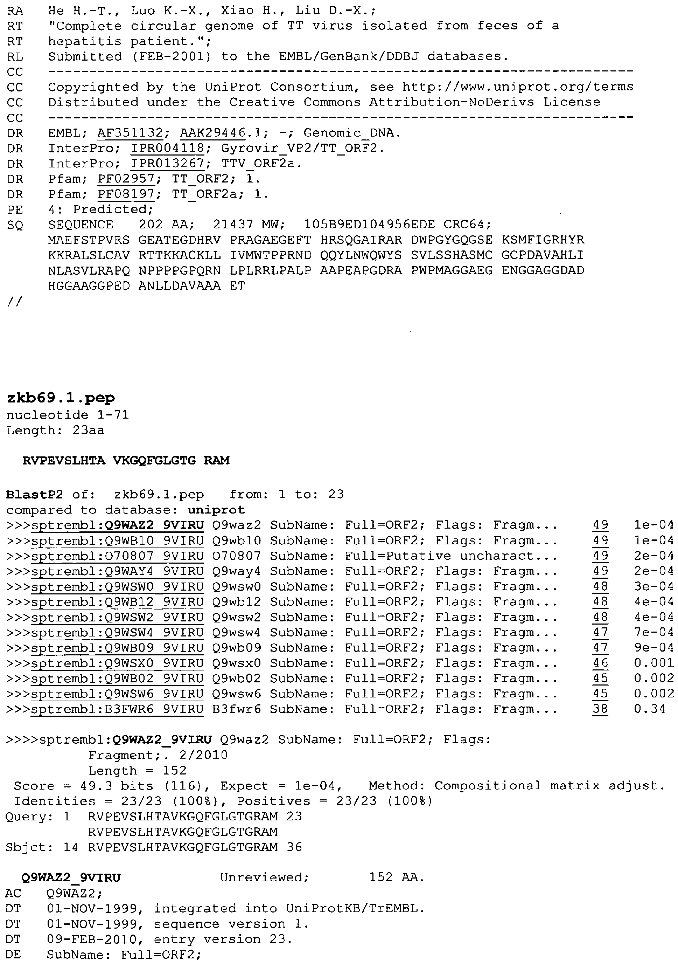

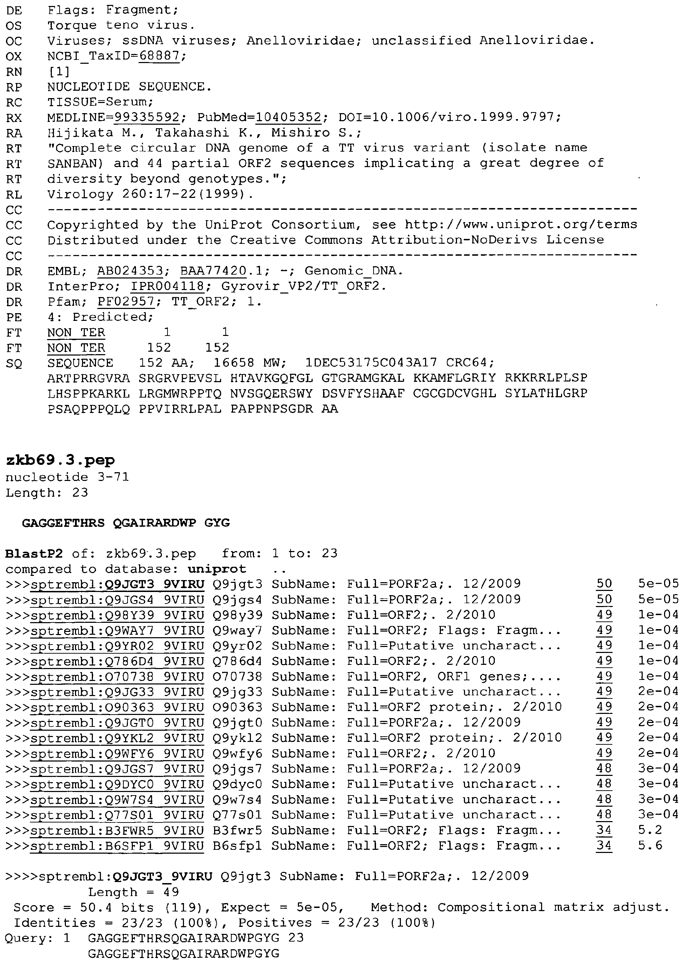

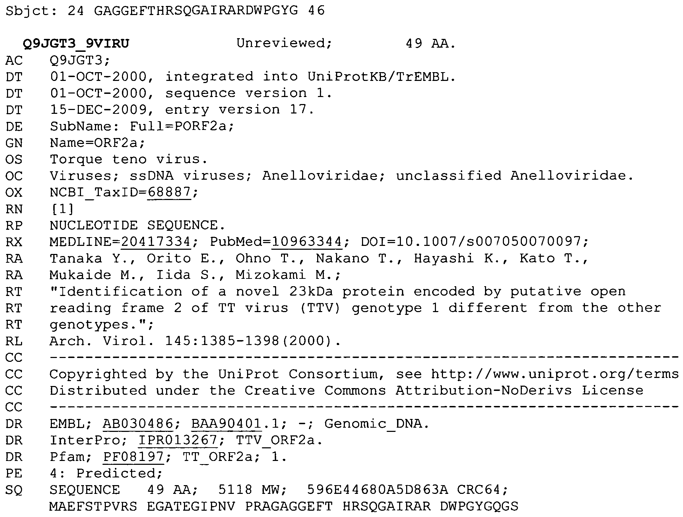

Figure 14: Open reading frames (ORFs) found within the nucleotide sequence of 71 nt
   zyb2.1.pep, zyb9.1.pep, and zkb69.1.pep are starting at the first triplet, zyb2.3.pep, zyb9.3.pep, zkb5.3.pep, and zkb69.3.pep are starting from the third triplet. This region is actively transcribed.

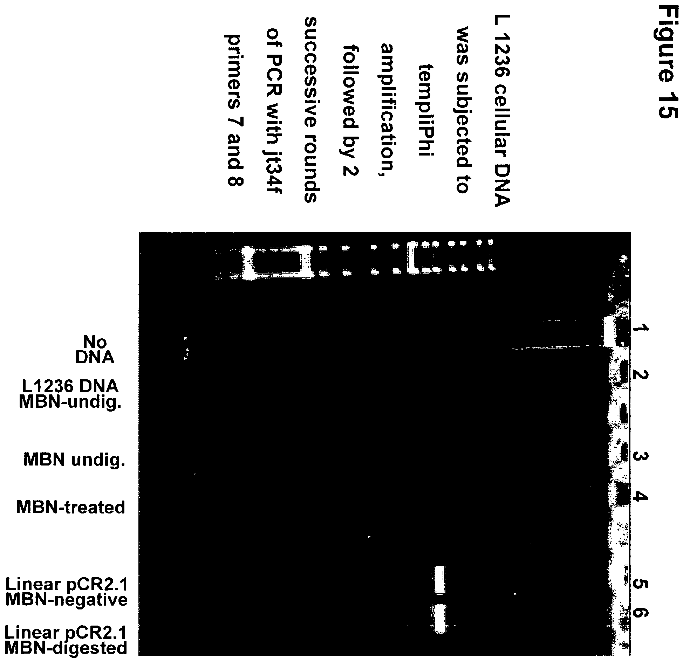

Figure 15: Digestion of single-stranded DNA by mung-bean nuclease (MBN) Lanes 2 and 3 show that the amplified DNA can be digested by pretreatment with MBN. Lanes 5 and 6 demonstrate that plasmid-DNA pretreated in the same way is not digested by MBN.

The present invention provides a TT virus polynucleic acid consisting of a nucleotide sequence shown in Figure 6 as defined in claim 1.

The person skilled in the art can easily determine which nucleic acid sequences are related to the nucleotide sequence of Figure 6 or which fragments are still capable of replicating autonomously by using standard assays or the assays described in the examples, below.

The present invention more specifically relates to a TT virus polynucleic acid having (a) a nucleotide sequence shown in Figure 6, or (b) the complement of (a)

The term "polynucleic acid" refers to a single-stranded or double-stranded nucleic acid sequence. A polynucleic acid may consist of deoxyribonucleotides or ribonucleotides, nucleotide analogues or modified nucleotides, or may have been adapted for therapeutic purposes. The TT virus polynucleic acid is a single-stranded DNA.

The TT virus polynucleic acid of the invention might be present as an extrachromosomal episome, might be integrated into the host's genome and/or might be linked to a host cell DNA, e.g., a DNA comprising a growth-stimulating host cell gene, oncogene or containing truncated host cell genes with altered immunogenicity.

Preferably, the TT virus polynucleic acid of the invention comprises a nucleotide sequence being selected from the group of nucleotide sequences shown in Figures 8 and 9.

TTV polynucleic acid sequences which are similar to the sequences as shown in Figure 6 can be characterized and isolated according to any of the techniques known in the art, such as amplification by means of sequence-specific primers, hybridization with sequence-specific probes under more or less stringent conditions, sequence determination of the genetic information of TTV, etc.

The present invention relates to a recombinant expression vector comprising a TTV polynucleic acid of the invention as defined above operably linked to prokaryotic or eukaryotic transcription and translation control elements.

The term "vector" may comprise a plasmid, a cosmid, an artificial chromosome, a phage, or a virus. Particularly useful for vaccine development may be TT virus recombinant molecules, BCG or adenoviral vectors, as well as avipox recombinant viruses.

The term "recombinantly expressed" used within the context of the present invention refers to the fact that the polypeptides of the present invention are produced by recombinant expression methods be it in prokaryotes, or lower or higher eukaryotes as discussed in detail below.

The term "lower eukaryote" refers to host cells such as yeast, fungi and the like. Lower eukaryotes are generally (but not necessarily) unicellular. Preferred lower eukaryotes are yeasts, particularly species within Saccharomyces, Schizosaccharomyces, Kluiveromyces, Pichia (e. g. Pichia pastoris), Hansenula (e. g. Hansenula polymorph), Schwaniomyces, Schizosaccharomyces, Yarowia, Zygosaccharomyces and the like. Saccharomyces cerevisiae, S. carlsbergensis and K. lactis are the most commonly used yeast hosts, and are convenient fungal hosts.

The term "higher eukaryote" refers to host cells derived from higher animals, such as mammals, reptiles, insects, and the like. Presently preferred higher eukaryote host cells are derived from Chinese hamster (e. g. CHO), monkey (e. g. COS and Vero cells), baby hamster kidney (BHK), pig kidney (PK15), rabbit kidney 13 cells (RK13), the human osteosarcoma cell line 143 B, the human cell line HeLa and human hepatoma cell lines like Hep G2, and insect cell lines (e.g. Spodoptera frugiperda). The host cells may be provided in suspension or flask cultures, tissue cultures, organ cultures and the like.

The term "prokaryotes" refers to hosts such as E. coli, Lactobacillus, Lactococcus, Salmonella, Streptococcus, Bacillus subtilis or Streptomyces. Also these hosts are contemplated within the present invention.

The term "host cell" refers to cells which can be or have been, used as recipients for a recombinant vector or other transfer polynucleotide, and include the progeny of the original cell which has been transfected. It is understood that the progeny of a single parental cell may not necessarily be completely identical in morphology or in genomic or total DNA complement as the original parent, due to natural, accidental, or deliberate mutation or recombination.

The term "replicon" is any genetic element, e. g., a plasmid, a chromosome, a virus, a cosmid, etc., that behaves as an autonomous unit of polynucleotide replication within a cell, i. e., capable of replication under its own control.

The term "vector" is a replicon further comprising sequences providing replication and/or expression of a desired open reading frame.

The term "control element" refers to polynucleotide sequences which are necessary to effect the expression of coding sequences to which they are ligated. The nature of such control sequences differs depending upon the host organism; in prokaryotes, such control sequences generally include promoter, ribosomal binding site, splicing sites and terminators; in eukaryotes, generally, such control sequences include promoters, splicing sites, terminators and, in some instances, enhancers. The term "control elements" is intended to include, at a minimum, all components whose presence is necessary for expression, and may also include additional components whose presence is advantageous, for example, leader sequences which govern secretion.

The term "promoter" is a nucleotide sequence which is comprised of consensus sequences which allow the binding of RNA polymerase to the DNA template in a manner such that mRNA production initiates at the normal transcription initiation site for the adjacent structural gene.

The expression "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

The segment of the TTV DNA encoding the desired sequence inserted into the vector sequence may be attached to a signal sequence. Said signal sequence may be that from a non-TTV source, but particularly preferred constructs according to the present invention contain signal sequences appearing in the TTV genome before the respective start points of the proteins.

Higher eukaryotes may be transformed with vectors, or may be infected with a recombinant virus, for example a recombinant vaccinia virus. Techniques and vectors for the insertion of foreign DNA into vaccinia virus are well known in the art, and utilize, for example homologous recombination. A wide variety of viral promoter sequences, possibly terminator sequences and poly(A)-addition sequences, possibly enhancer sequences and possibly amplification sequences, all required for the mammalian expression, are available in the art. Vaccinia is particularly preferred since vaccinia halts the expression of host cell proteins. For vaccination of humans the avipox and Ankara Modified Virus (AMV) are particularly useful vectors.

Also known are insect expression transfer vectors derived from baculovirus Autographa californica nuclear polyhedrosis virus (AcNPV), which is a helper-independent viral expression vector. Expression vectors derived from this system usually use the strong viral polyhedrin gene promoter to drive the expression of heterologous genes. Different vectors as well as methods for the introduction of heterologous DNA into the desired site of baculovirus are available to the man skilled in the art for baculovirus expression. Also different signals for posttranslational modification recognized by insect cells are known in the art.

The present invention also relates to a host cell as defined above comprising a recombinant vector as defined above.

Preferred vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR.

Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957. The TTV polynucleotide sequences of the invention may also serve as a suitable vector itself composed solely of chimeric TTV host cell DNA sequences. In addition, the nucleotide sequences of the invention may be used for the construction of artificial chromosomes.

The following examples are intended to illustrate, but not to limit the invention. While such Examples are typical of those that might be used, other methods known to those skilled in the art may alternatively be utilized.

### Example 1

### Demonstration of the persistence of TTV DNA in cells from tissue culture lines derived from malignant tumors

Cell lines derived from malignant tumors possess one advantage over primary tumor biopsy material. They commonly represent pure preparations of cancer cells, whereas primary materials are commonly contaminated by normal mesenchymal cells, by cells of the hematopoietic system and normal epithelial cells. On the other hand, one disadvantage of tissue culture lines may arise from the selection of specific clones growing under tissue culture conditions and the acquisition of secondary genetic modifications in the course of long-term cultivation. In addition, fetal calf sera may pose a risk due to the introduction of cattle viruses which survive serum inactivation procedures (e.g. bovine polyomavirus); see Table 1 summarizing these advantages/disadvantages.

**Table 1**

| **Analysis of primary tumor biopsies vs established cell lines for TTV-related sequences** | | | |
|---|---|---|---|
| | | | |
| **Biopsies** | | **Cell lines** | |
| **Advantage** | **Disadvantage** | **Advantage** | **Disadvantage** |
| **Authentic materials** | **Contaminated by admixture of normal cells** | **Pure preparations of cancer cells** | **Selection of specific Clones adapted to tissue culture conditions** |
| | **Search for TTV sequences clouded by the uniform presence of TTV in the peripheral blood** | **Available in unlimited amounts** | **Secondary genetic changes during long-term cultivation** |
| | **Availability limited** | | **Use of fetal calf serum poses the risk of contaminations with cattle viruses** |

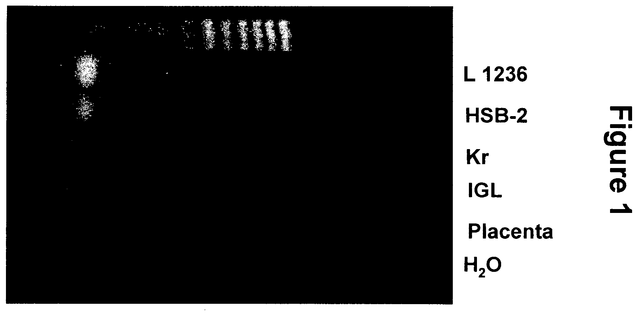

Attempts to find TTV DNA in human primary tumor materials suffers from one disadvantage: the plurality of TTV genotypes in human material (8). This renders it virtually impossible to identify a specific genotype as an etiologic agent for a human cancer type. For these reasons studies on the persistence of TTV DNA sequences in cells derived from cancer tissue culture lines were initiated. Thus far the results have been extremely surprising: PCR primers used to discover regions of the TTV large open reading frame have been entirely unsuccessful. However, other primer combinations, discovering exclusively a short GC-rich regulatory region of the TTV genome of about 71 bases, detected this sequence in a larger number of cell lines (Figure 1). This regulatory region is highly conserved among different TTV genotypes and is not present in the human genome data bank.

In a first series of experiments the same sequence was discovered in a number of additional cell lines. These included the following lines:
- *MCF7* (breast cancer line);
- *HAK-1, KMH-2, L1236* (all Epstein-Barr virus negative Hodgkin's lymphoma lines);
- *Y69* (Epstein-Barr virus negative B-lymphoma)
- *HSB-2* (acute lymphocytic leukemia);
- *P3HR-1* (Epstein-Barr virus-positive Burkitt's lymphoma);
- *BJAB* (Epstein-Barr virus negative Burkitt's lymphoma);
- *Ng* (EBV-immortalized B lymphoblasts from a patient with multiple sclerosis)-
-

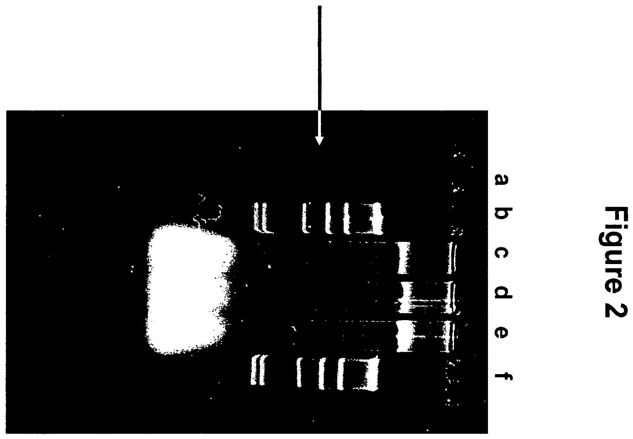

Besides these 9 positive lines, two melanoma cell lines (IGL and KR, Fig. 1) and human placenta DNA were negative in initial experiments. Interestingly, after removal of spooled DNA from L1236 cells and RNase treatment of the remaining solution, besides mitochondrial DNA two faint bands of similar size became visible banding between positions 4.3-6.6 kb (double-stranded DNA size marker) in the agarose gels (Fig. 2). Analysis of these sequences revealed again the presence of the TTV regulatory region. Mung-bean nuclease, digesting selectively single-stranded DNA, completely abolished the cellular DNA-containing bands from four multiple sclerosis biopsies in contrast to double-stranded control DNA, underlining the single-stranded nature of the former. Similar studies are presently conducted for isolates from tumor DNA.

### Example 2

### Analyses of chimeric TTV/truncated host cell DNA sequences

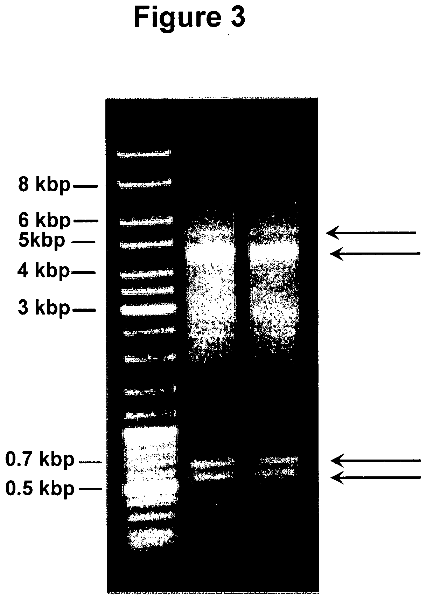

Initially, all attempts failed to use primers in outwards orientation starting within the regulatory region in order to find flanking TT viral DNA, surrounding this region. Invariably, however, human cellular DNA was demonstrated in the respective clones (Fig. 3).

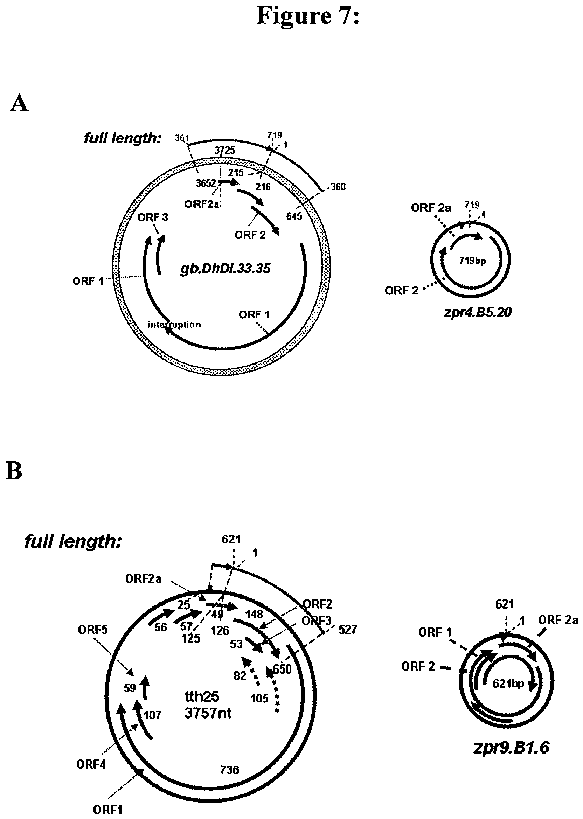

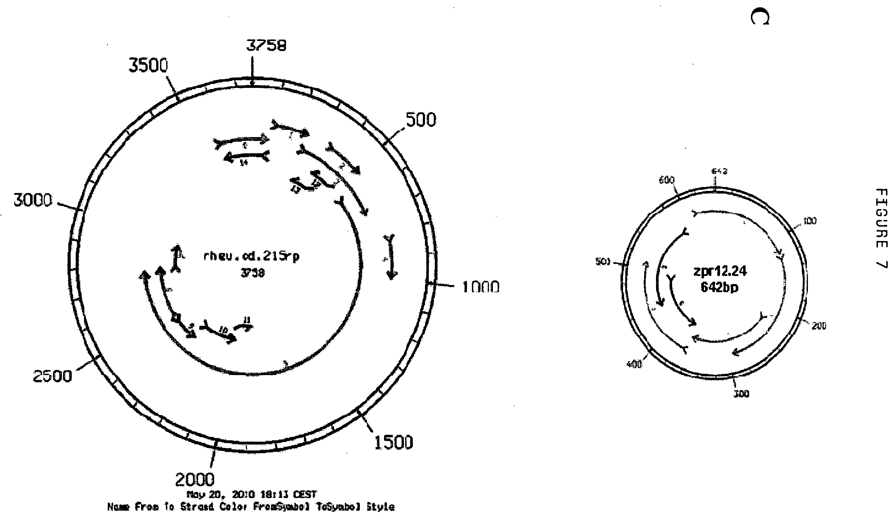

The human genes in these clones and their arrangements within the single-stranded episomal DNA, obviously controlled by the TTV 71 base region, are presently being analyzed. The available data indicate a substantial variation in the uptake of commonly truncated host cell genes. Their possible conversion into growth-stimulating oncogenes or into functions interfering with tumorsuppressor genes requires functional tests which are presently under investigation. The same accounts for rearranged TTV virus sequences. Some of the available data are presented in Figures 7, 8, 9, and 11 to 13.

### List of References

1. Nishizawa, T., Okamoto, H., Kato, N. et al. A novel DNA virus (TTV) associated with elevated transaminase levels in posttransfusion hepatitis of unknown etiology. Biochem. Biophys. Fes. Commun. 241: 92-97, 1997.
2. Okamoto, H. TT viruses in animals. Review. In: TT Viruses, the still Elusive Pathogens (de Villiers, E.-M., zur Hausen, H. eds.), Curr. Topics Microbiol. Immunol. E.-M. de Villiers, H. zur Hausen (Eds), Vol. 331: 35-52, 2009.
3. Biagini, P., Todd, D., Bendinelli, M. et al., Anellovirus. In: Virus Taxonomy, VIIIth Report of the International Committee for the Taxonomy of Viruses. London: Elsevier/ Academic Press pp. 335-341, 2005.
4. Gerner, P., Oettinger, R., Gerner, W., et al. Mother-to-infant transmission of TT virus: prevalence, extent and mechanism of vertical transmission. Pediatr. Infect. Dis. J. 19: 1074-1077, 2000.
5. Goto, K., Sugiyama, K., Ando, T., e al., Detection rates of TT virus DNA in serum of umbilical cord blood, breast milk and saliva. Tohoku J., Exp.Med. 191: 203-207, 2000.
6. Borkosky, S., Whitley, C., and de Villiers, E.-M., unpublished results.
7. Leppik, L., Gunst, K., Lehtinen, M., et al., In vivo and in vitro intragenomic rearrangement of TT viruses. J. Virol. 81: 9346-9356, 2007.
8. Jelcic, I., Hotz-Wagenblatt, A., Hunzicker, A., et al. Isolation of multiple TT virus genotypes from spleen biopsy tissue from a Hodgkin's disease patient: genome reorganization and diversity of the hypervariable region. J. Virol. 78: 7498-7507, 2004.
9. de Villiers, E.-M., Schmidt, R., Delius, H., et al. Heterogenetity of TT virus related sequences isolated from human tumour biopsy specimens. J. Mol. Med. 80: 44-50, 2002.
10. de Villiers, E.-M., Bulajic, M., Nitsch, C., et al. TTV infection in colorectal cancer tissues and normal mucosa. Int. J.Cancer 121: 2109-2112., 2007.
11. de Villiers, E.M., Kimmel, R., Leppik, L., and Gunst, K. Intragenomic rearrangements in TT viruses; a possible role in the pathogenesis of disease. In: TT Viruses, the still Elusive Pathogens (de Villiers, E.-M., zur Hausen, H. eds.), Curr. Topics Microbiol. Immunol. 331: 91-107, 2009.
12. Matsukara, M., Shinozuka, K., Zon, G., Mitsuya, H., Reitz, M., Cohen, J., Broder, S. Proc. Natl. Acad. Sci. USA 84(21): 7706-10 (1987).
13. Miller, P., Yano, J., Yano, E., Carroll, C., Jayaram, K., Ts'o, P. (1979) Nonionic nucleic acid analogues. Synthesis and characterization of dideoxyribonucleoside methylphosphonates. Biochemistry 18(23): 5134-43.
14. Nielsen, P., Egholm, M., Berg, R., Buchardt, O. (1991) Sequence-selective recognition of DNA by strand displacement with a thymine-substituted polyamide. Science 254(5037): 1497-500.
15. Nielsen, P., Egholm, M., Berg, R., Buchardt, O. (1993) Sequence specific inhibition of DNA restriction enzyme cleavage by PNA. Nucleic-Acids-Res. 21(2): 197-200.
16. Asseline, U., Delarue, M., Lancelot, G., Toulme, F., Thuong, N. (1984) Nucleic acid-binding molecules with high affinity and base sequence specificity: intercalating agents covalently linked to oligodeoxynucleotides. Proc. Natl. Acad. Sci. USA 81(11): 3297-301.
17. Kwok, S., Kellogg, DE., McKinney, N., Spasic, D., Goda, L., Levenson, C., Sninsky, JJ. (1990) Effects of primer-template mismatches on the polymerase chain reaction: human immunodeficiency virus type 1 model studies. Nucleic Acids Res 18: 999-1005.
18. Landgren, U., Kaiser, R., Sanders, J., Hood, L. (1988) A ligase-mediated gene detection technique. Science 241:1077-1080.
19. Wu, D., Wallace, B. (1989) The ligation amplification reaction (LAR)-amplification of specific DNA sequences using sequential rounds of template-dependent ligation. Genomics 4: 560-569.
20. Walker, GT., Fraiser, MS., Schram, JL., Little, MC., Nadeau, JG., Malinowski, DP. (1992) Strand displacement amplification -- an isothermal, in vitro DNA amplification technique. Nucleic Acids Res 20: 1691-6.
21. Guatelli, J., Whitfield, K., Kwoh, D., Barringer, K., Richman, D., Gengeras, T. (1990) Isothermal, in vitro amplification of nucleic acids by a multienzyme reaction modeled after retroviral replication. Proc Natl Acad Sci USA 87 : 1874-1878.
22. Compton, J. (1991) Nucleic acid sequence-based amplification. Nature 350: 91-92.
23. Kwoh, DY., Davis, GR., Whitfield, KM., Chappelle, HL., DiMichele, LJ., Gingeras, TR. (1986) Transcription-based amplification system and detection of amplified human immunodeficiency virus type 1 with a bead-based sandwich hybridization format. Proc Natl Acad Sci USA, 86: 1173-7.
24. Duck, P. (1990) Probe amplifier system based on chimeric cycling oligonucleotides. Biotechniques 9: 142-147.
25. Lizardi, PM., Kramer, FR. (1991) Exponential amplification of nucleic acids: new diagnostics using DNA polymerases and RNA replicases. Trends Biotechnol 19: 53-8.
26. Lomeli, H., Tyagi, S., Pritchard, CG., Lizardi, PM., Kramer, FR. (1989) Quantitative assays based on the use of replicatable hybridization probes. Clin Chem 35: 1826-31.
27. Köhler et al., Nature 256 (1975), 495.
28. Wahl et al., J. Nucl. Med. 24: 316-325 (1983).
29. Jacobs, KA. et al. (1988) The thermal stability of oligonucleotide duplexes is sequence independent in tetraalkylammonium salt solutions: application to indeifying recobinant DNA clones. Nucleic Acids Res 16:4637-50.
30. Zimmermann et al., (1994) Neuron 12, 11-24.
31. Vidal et al.; (1990) EMBO J. 9, 833-840.
32. Mayford et al., (1995), Cell 81, 891-904.
33. Pinkert et al., (1987) Genes & Dev. 1, 268-76).
34. Nielsen et al., Science 254 (1991), 1497-1500.
35. Fingl et al., The Pharmocological Basis of Therapeutics, Goodman and Gilman, eds. Macmillan Publishing Co., New York, pp. 1-46 (1975).

## Claims

1. An expression vector which is capable of replicating autonomously in a human host cell comprising a TT virus polynucleic acid operably linked to
(i) prokaryotic transcription and translation control elements, wherein the prokaryotic control elements comprise a promoter, a ribosomal binding site, splicing sites and a terminator, or
(ii) eukaryotic transcription and translation control elements, wherein the eukaryotic control elements comprise a promoter, splicing sites and a terminater and
the transcription and translation control elements control the expression of the TT virus polynucleic acid consisting of
(a) a nucleotide sequence selected from the group consisting of the sequences shown in Figure 6 and coding one of the peptides designated as zyb2.1.pep, zyb9.1.pep, zkb69.1.pep, zyb2.3.pep, zyb9.3.pep, zkb5.3.pep and zkb69.3.pep shown in Figure 14;
or
(b) a nucleotide sequence which is the complement of the nucleotide sequence of (a).

2. The vector of claim 1, wherein the vector comprises a chimeric TT virus polynucleic acid of the TT virus polynucleic acid as defined in claim 1 and a cellular nucleic acid, the chimeric TT virus polynucleic acid is operably linked to prokaryotic or eukaryotic transcription and translation control elements and the cellular nucleic acid has a nucleotide sequence being selected from the group of nucleotide sequences consisting of gbDhDi38 shown in Figure 8A, gb40.27 shown in Figure 8B, gb43.30 shown in Figure 8C, hod11 shown in Figure 9A, hoht33 shown in Figure 9B and hoht22 shown in Figure 9C.

3. The expression vector of claim 1 or 2, wherein the vector is selected from the group consisting of plasmid, cosmid, and artificial chromosome.

4. A host cell comprising an expression vector according to any one of claims 1-3.

## Patentansprüche

1. Expressionsvektor, der in der Lage ist, autonom in einer menschlichen Wirtszelle zu replizieren, umfassend eine TT-Virus Polynukleinsäure, die funktionsfähig verknüpft ist mit
(i) prokaryotischen Transkriptions- und Translationskontrollelementen, wobei die prokaryotischen Kontrollelemente einen Promotor, eine ribosomale Bindungsstelle, Spleißstellen und einen Terminator umfassen, oder
(ii) eukaryotischen Transkriptions- und Translationssteuerelementen, wobei die eukaryotischen Kontrollelemente einen Promotor, Spleißstellen und einen Terminator umfassen und
die Transkriptions- und Translationskontrollelemente die Expression der TT-Virus Polynukleinsäure bestehend aus
(a) einer Nukleotidsequenz, ausgewählt aus der Gruppe bestehend aus den in Fig. 6 gezeigten Sequenzen, und für eines der Peptide kodiert, die als zyb2.1.pep, zyb9.1.pep, zkb69.1.pep, zyb2.3.pep, zyb9.3.pep, zkb5.3.pep und zbk69.3.pep bezeichnet werden und in Abbildung 14 gezeigt sind;
oder
(b) einer Nukleotidsequenz, die das Komplement der Nukleotidsequenz von (a) ist, kontrollieren.

2. Vektor nach Anspruch 1, wobei der Vektor eine chimäre TT-Virus Polynukleinsäure einer TT-Virus Polynukleinsäure, wie in Anspruch 1 definiert, und einer zellulären Nukleinsäure umfasst, wobei die chimäre TT-Virus Polynukleinsäure funktionell mit prokaryotischen oder eukaryotischen Transkriptions- und Translationskontrollelementen verknüpft ist und die zelluläre Nukleinsäure eine Nukleotidsequenz hat, die aus der Gruppe von Nukleotidsequenzen ausgewählt ist, die aus gbDhDi38 wie in Abbildung 8A gezeigt, gb40.27 wie in Abbildung 8B gezeigt, gb43.30 wie in Abbildung 8C gezeigt, hod11 wie in Abbildung 9A gezeigt, hoht33 wie in Abbildung 9B gezeigt und hoht22 wie in Abbildung Abbildung 9C gezeigt, besteht.

3. Expressionsvektor nach Anspruch 1 oder 2, wobei der Vektor ausgewählt ist aus der Gruppe bestehend aus Plasmid, Cosmid und künstlichem Chromosom.

4. Wirtszelle, umfassend einen Expressionsvektor nach einem der Ansprüche 1 bis 3.

## Revendications

1. Vecteur d'expression qui est capable de se répliquer de manière autonome dans une cellule hôte humaine comprenant un acide polynucléique du virus TT fonctionnellement lié à
(i) des éléments de régulation de transcription et de traduction procaryotes, où les éléments de régulation procaryotes comprennent un promoteur, un site de liaison ribosomique, des sites d'épissage et un terminateur, ou
(ii) des éléments de régulation de transcription et de traduction eucaryotes, où les éléments de régulation eucaryotes comprennent un promoteur, des sites d'épissage et un terminateur et
les éléments de régulation de transcription et de traduction régulent l'expression de l'acide polynucléique du virus TT consistant en
(a) une séquence nucléotidique sélectionnée dans le groupe composé des séquences représentées à la figure 6 et codant pour l'un des peptides désignés par zyb2.1.pep, zyb9.1.pep, zkb69.1.pep, zyb2.3.pep, zyb9.3.pep, zkb5.3.pep et zkb69.3.pep représentés à la figure 14; ou
(b) une séquence nucléotidique qui est le complément de la séquence nucléotidique de (a).

2. Vecteur selon la revendication 1, dans lequel le vecteur comprend un acide polynucléique du virus TT chimérique de l'acide polynucléique du virus TT selon la revendication 1 et un acide nucléique cellulaire, l'acide polynucléique du virus TT chimérique est fonctionnellement lié à des éléments de régulation de transcription et de traduction procaryotes ou eucaryotes et l'acide nucléique cellulaire présente une séquence nucléotidique sélectionnée dans le groupe de séquences nucléotidiques composé de gbDhDi3B représentée à la figure 8A, gb40.27 représentée à la figure 8B, gb43.30 représentée à la figure 8C, hod11 représentée à la figure 9B et hoht22 représentée à la figure 9C.

3. Vecteur d'expression selon la revendication 1 ou 2, dans lequel le vecteur est sélectionné dans le groupe composé de plasmide, de cosmide et de chromosome artificiel.

4. Cellule hôte comprenant un vecteur d'expression selon l'une quelconque des revendications 1 à 3.
